# EUROPEAN PATENT APPLICATION

(11) **EP 3 854 385 A1**
(43) Date of publication of application: **28.07.2021**
(21) Application number: 20153818.8
(22) Date of filing: 27.01.2020
(51) Int. Cl.: A61K 9/20, A61K 9/48, A61K 31/519, A61P 17/06, A61P 19/02, A61P 35/02, A61K 9/28

(54) **METHOTREXATE DOSAGE FORM**

(71) Applicant: ROS Therapeutics ApS, 2200 Copenhagen (DK)
(72) Inventor: Højgaard, Bent, 3450 Allerød (DK); Damgaard Jensen, Hanne, 4000 Roskilde (DK)
(74) Representative: Poulsen, Niels Jakob

(57) **Abstract**

The present invention relates to a pharmaceutical dosage form comprising an active ingredient such as methotrexate or a pharmaceutically acceptable salt thereof, in particular in the form of pellets, such as multiparticulates, mini-tablets or granulate. It further relates to oral dosage forms for which saturation kinetics limits the oral use of higher dosages of active ingredients.

## Description

The present invention relates to a pharmaceutical dosage form comprising an active ingredient such as methotrexate or a pharmaceutically acceptable salt thereof, in particular in the form of pellets, including multiparticulates, mini-tablets and granulate. It further relates to oral dosage forms for which saturation kinetics limits the use of higher dosages of active ingredients.

### Technical Background

Methotrexate oral formulations in the form of tablets are common on the market. Tablets comprising 2.5 mg methotrexate are sold by the companies TEVA, Sandoz and others.

According to Goodman et al. (Goodman et al, Outcomes Related to Methotrexate Dose and Route of Administration in Patients with Rheumatoid Arthritis: A Systematic Literature Review, Clin Exp Rheumatol, 2015) efficacy and toxicity of methotrexate appear related to absorbed dose of methotrexate, not to route of administration. While bioavailability is greater for parenteral methotrexate, there is no evidence yet that splitting the oral dose of methotrexate is less advantageous, safer or more tolerable than administering parenteral methotrexate. However, it appears to be beneficial to treat with higher doses of methotrexate, initially, and subsequently to switch to parenteral administration of methotrexate when the clinical response to an oral dose becomes inadequate.

According to Taylor et al (Taylor et al, How to Get the Most from Methotrexate (MTX) Treatment for Your Rheumatoid Arthritis Patients? - MTX in the Treat-to-Target Strategy", Journal of Clinical Medicine, 2019) parenteral administration of methotrexate has the advantages of maximizing bioavailability, reducing gastrointestinal intolerance and potentially enhancing compliance and adherence.

The international patent application WO2007040997 describes an invention that relates to a pharmaceutical drug delivery system for the controlled release of absorption window active agents which have an absorption window in the gastrointestinal tract, i.e. are usually absorbed in the duodenum and/or jejunum or have a site of treatment in or proximal to the gastrointestinal tract or degrade in the colon.

The controlled-release dosage forms may provide good bioavailability of absorption window active agents. The document describes that anti-tumor agents suitable for the invention includes 5- cisplatin, doxorubicin, etoposide, fluorouracil, methotrexate, mitomycin, semustine, or mixtures of any thereof.

### Summary of the invention

For certain active ingredients, having a narrow therapeutic index such as methotrexate, the side effects have to be balanced very carefully against the efficacy upon choosing the administration route and administered dosage. For such active ingredients it is desirable to be able to vary the dosage for each individual patient. However, for some active ingredients, such as methotrexate, the use is further restricted by saturation kinetics. Increasing the amount of active ingredient in a single dosage form may only provide little or no improved clinical effect, due to saturation of active transport mechanisms from the gut lumen and into the blood stream, while increasing the side effects, due to uptake in non-target cells. For such active ingredients it may be desirable, not only to be able to vary the amount of active ingredient in a dosage form, but also to change the release profile, e.g. of an oral dosage form, according to the amount of active ingredient in the dosage form. For the industrial manufacture of drugs, it is expensive and cumbersome to produce a large number of different pharmaceutical formulations with different strengths and release characteristics. It is envisaged that the present concept may also be used with other active ingredients, such as chemotherapeutic agents.

Upon comparing subcutaneous and oral administration of methotrexate, oral administration of an immediate release formulation suffers from the drawback that saturation kinetics becomes prevalent when single dosages are increased above about 10-15 mg.

There is a need for an oral pharmaceutical composition with increased bioavailability of methotrexate at higher dose levels, such as 15 mg and more. Further, there is a need for an oral pharmaceutical composition with dose proportional increase in bioavailability of methotrexate at a dose level of 15 mg and more. In addition, there is a need for an oral pharmaceutical composition giving the patient the choice to spread the content of the composition on a meal, thus easing the administration. There is also a need for an easier method of manufacturing of a pharmaceutical product that is expected to be marketed in many different strengths.

Mini-tablet formulations are developed with immediate release profiles and with lag time/burst release profiles to deliver the dose in the intestine within maximum 4 hours from dosing. These different formulations can then be combined in a capsule in an attempt to increase bioavailability compared to conventional tablets.

Methotrexate exists as either the acid (molecular weight: 454.4 g/mol) or the di-sodium salt (molecular weight: 498.40 g/mol). In pharmaceutical products, methotrexate is declared as the acid, but often added as the di-sodium salt (conversion factor 1.096), as this is freely soluble in water; the acid is practically insoluble in water. Here, in the case of doubt, the term "methotrexate" refers to methotrexate as well as pharmaceutically acceptable salts thereof.

Methotrexate is also known with names and formulas such as N-[4-[[2,4-Diamino-6-pteridinyl)methyl]methylamino]benzoyl]-L-glutamic acid; 4-amino-N¹⁰-methylpteroylglutamic acid, 4-amino-10-methylfolic acid, methylaminopterin, amethopterin, MTX, and C₂₀H₂₂N₈O₅.

In certain embodiments, the pharmaceutical composition is made by the technology as found in the international patent application WO2018011181, all of which is incorporated by reference in its entirety.

The international patent application WO2018011181 in the name of Contera Pharma and Solural Pharma describes a pulsatile drug delivery system that enables a delayed burst release of levodopa and DOPA decarboxylase inhibitors including carbidopa in the small intestine, thereby providing for improved management of morning akinesia in Parkinson's disease patients. The application comprises an aspect to provide a pulsatile release pharmaceutical composition comprising a. levodopa and a DOPA decarboxylase inhibitor, and b. a pulsatile release component providing for a predetermined lag time followed by a pulse release of said levodopa and said DOPA decarboxylase inhibitor. The application further describes an aspect to provide a pulsatile release pharmaceutical composition comprising, separately or together, a. a first pulsatile release component comprising levodopa, said first pulsatile release component providing for a predetermined lag time followed by a pulse release of levodopa, and b. a second pulsatile release component comprising a DOPA decarboxylase inhibitor, said second pulsatile release component providing for a predetermined lag time followed by a pulse release of said DOPA decarboxylase inhibitor. Minitablets comprising 2.8, 3.0, 4.0 mg levodopa and minitablets comprising 2.6 mg Carbidopa were described. 33 Levodopa mini-tablets coated to 25% weight gain and ten film coated Carbidopa mini-tablets were mixed and filled into a hard-shell gelatin capsule size 0. The capsule holds a dose of 100mg levodopa + 25mg Carbidopa and the active component will be released after a lag-time; Carbidopa will be released followed by Levodopa.

According to an aspect, the invention concerns a pharmaceutical composition comprising a plurality of pellets, wherein each pellet has a smallest diameter ≤ 5 mm, preferably ≤ 3 mm, and wherein each pellet comprises methotrexate or a pharmaceutically acceptable salt thereof, or no methotrexate, and/or another active ingredient.

A pharmaceutical composition is preferably a pharmaceutically acceptable composition.

A "pellet" may be defined as a rounded, spherical or cylindrical body or medicine. The term "pellet" may further comprise a small ball or tube-shaped piece of any substance. A "pellet" may also refer to a small tablet, i.e. a mini tablet, granulate, or multiparticulates. A pellet of the present invention is preferably spheronized or spherical, but may in principle have another suitable shape.

The term "smallest diameter" means that the pellet is able to pass through a circular aperture with this diameter, e.g. in the case of a cylindrical body it may be the diameter of the body, and the term diameter does not necessarily imply that the pellet is spherical.

The pyloric sphincter is a band of smooth muscle at the junction between the pylorus of the stomach and the duodenum of the small intestine. It plays an important role in digestion, where it acts as a valve to control the flow of partially digested food from the stomach to the small intestine. Liquids and smaller items get transported quickly through the pyloric sphincter. Larger items get transported during digestion, when the sphincter relaxes intermittently and aperture opens. Thus, a small pharmaceutical composition, preferably having a diameter ≤ 3 mm, may be emptied from the stomach independent of gastric emptying and transported to the duodenum irrespective of whether the subject has been eating or not.

According to another aspect, the invention concerns a pharmaceutical composition comprising a plurality of pellets, wherein each pellet comprises an active ingredient such as methotrexate or a pharmaceutically acceptable salt thereof, and preferably wherein at least one of the pellets provides immediate release of said active ingredient, such as methotrexate; and preferably wherein at least one of the pellets provides delayed release and/or a predetermined lag time prior to release of said active ingredient for dosages at 15 mg or above; and wherein at least 80 % of the total amount of methotrexate of said pharmaceutical composition have been released within 4 hours of administration and/or as measured in an USP Dissolution Apparatus 2 in 500 ml 0.1 N HCI at 37°C ± 0.5°C.

The total amount of released methotrexate after administration may preferably be measured in an USP Dissolution Apparatus 2, paddle (37°C ± 0.5°C).

According to another aspect, the invention concerns a pharmaceutical composition comprising
i. At least one pellet providing immediate release of methotrexate, and
ii. At least one pellet providing delayed release of methotrexate for total dosages of the composition of 15 mg or more;

Wherein said pharmaceutical composition allow release of at least 80 % of the total methotrexate within 4 hours of administration.

According to another aspect, the invention concerns a pharmaceutical composition comprising a plurality of pellets, said composition comprising a total dosage of 1 - 30 mg methotrexate, and wherein said composition comprises:
a. At least one pellet A providing immediate release of methotrexate.
b. For the composition comprising at least 5 mg, preferably 7.5 mg, more preferred 10 mg methotrexate, preferably 12.5 mg, more preferred 15 mg, further at least one pellet B providing delayed release of methotrexate, and preferably
c. For the composition comprising at least 15 mg, preferred 17.5 mg, more preferred 20 mg methotrexate, further at least one pellet C providing delayed release of methotrexate wherein said pellet C provides slower release than said pellet B.

Having at least two, preferably at least three pellets, in the pharmaceutical formulation, with different release characteristics allow the release of methotrexate to be closer to saturation conditions without reaching saturation in a subject.

Combining pellets with different release profiles may have the advantage from a manufacturing point of view that various release profiles may be obtained, e.g. for different dosages, by combining a few different types of pellets. As an example, 30 different release profiles for 30 different dosages may be obtained by mixing two or three different types of pellet. This provides a simplified production process as compared to the situation where 30 different tablets with different strengths and release profiles have to be manufactured, each with a specific composition.

The ability to adjust the release profile to the strength of the dosage form allows for minimizing saturation kinetics and/or adverse effects and/or improving efficacy of the treatment.

According to an embodiment, pellet formulations are developed with immediate release and with lag time/burst release to deliver the dose in the intestine within maximum 4 hours from dosing. These different formulations can then be combined in a capsule. The combination of immediate release and delayed release of methotrexate may increase bioavailability compared to conventional tablets.

According to another aspect, the invention concerns a pharmaceutical composition comprising a plurality of at least two, preferably at least three, pellets, providing a total dosage of methotrexate of 1 - 30 mg, wherein said composition comprises pellets providing immediate release of methotrexate; and for dosages of 7.5 - 20 mg further comprises pellets providing delayed release of methotrexate; and for dosages of 15 - 30 mg comprises pellets providing an additional delayed release of methotrexate.

According to another aspect, the invention concerns a method for the manufacture of a pharmaceutical composition according to the invention.

According to another aspect, the invention concerns a method for the manufacture of a pharmaceutical composition according to the invention comprising:
i. Manufacturing at least two different types of pellets, wherein each type of pellet has specific release characteristics, and
ii. Mixing the pellets according to the desired final release characteristics and total amount of methothrexate.

According to another aspect, the invention concerns use of the pharmaceutical composition according to the invention in the manufacturing of a medicament for the treatment of a disease, wherein said disease is the disease according to the invention.

According to another aspect, the invention concerns a method of treating the disease according to the invention, comprising administering a therapeutically effective amount of the composition of the invention.

### Detailed Disclosure

According to an embodiment, the invention concerns a pharmaceutical composition comprising a plurality of pellets, wherein each pellet has a smallest diameter ≤ 5 mm, preferably ≤ 3 mm, and wherein each pellet comprises methotrexate or a pharmaceutically acceptable salt thereof.

According to an embodiment, the invention concerns a pharmaceutical composition comprising a plurality of pellets, wherein each pellet comprises methotrexate, and preferably wherein at least one of the pellets provides immediate release of methotrexate; and preferably wherein at least one of the pellets provides delayed release and/or a predetermined lag time prior to release of methotrexate; and wherein at least 80 % of the total amount of methotrexate of said pharmaceutical composition have been released within 4 hours of administration and/or as measured in an USP Dissolution Apparatus 2 in 500 ml 0.1 N HCl at 37°C ± 0.5°C.

The term "delayed release" refers to oral medicines that do not immediately disintegrate and release the active ingredient(s) into the body, and may also refer to burst release or extended release.

The term "immediate release" refers to oral medicines that release the active ingredient(s) into the body without delay.

The total amount of released methotrexate after administration may preferably be measured in an USP Dissolution Apparatus 2, paddle (37°C ± 0.5°C).

According to an embodiment the invention concerns a pharmaceutical composition comprising
i. At least one pellet providing immediate release of methotrexate, and
ii. At least one pellet providing delayed release of methotrexate;

Wherein said pharmaceutical composition allow release of at least 80 % of the total methotrexate within 4 hours of administration.

According to an embodiment the invention concerns a pharmaceutical composition comprising a plurality of pellets, said composition comprising a total dosage of 1 - 30 mg methotrexate, and wherein said composition comprises:
a. At least one pellet A providing immediate release of methotrexate.
b. For the composition comprising at least 15 mg, preferred 17.5 mg, more preferred 20 mg methotrexate, further at least one pellet B providing delayed release of methotrexate.
c. For the composition comprising at least 15 mg, preferred 17.5 mg, more preferred 20 mg methotrexate, further at least one pellet C providing delayed release of methotrexate wherein said pellet C provides slower release than said pellet B.

Combining pellets with different release profiles has the advantage from a manufacturing point of view that various release profiles may be obtained, e.g. for different dosages, by combining a few different types of pellets. As an example, 30 different release profiles for 30 different dosages may be obtained by mixing two or three different types of pellet, instead of having to manufacture 30 different tablets with different strengths and release profiles.

The ability to adjust the release profile to the strength of the dosage form allows for minimizing saturation kinetics and/or adverse effects and/or efficacy of the treatment.

According to an embodiment, pellet formulations are developed with immediate release and with lag time/burst release to deliver the dose in the intestine within maximum 4 hours from dosing. These different formulations can then be combined in a capsule. The combination of immediate release and delayed release of methotrexate may increase bioavailability compared to conventional tablets.

According to an embodiment, the invention concerns a pharmaceutical composition comprising a plurality of at least one, preferably two, and optionally at least three, pellets, providing a total dosage of methotrexate of 1 - 30 mg, wherein said composition comprises pellets providing immediate release of methotrexate; and for dosages of 7.5 - 20 mg further comprises pellets providing delayed release of methotrexate; and for dosages of 15 - 30 mg preferably comprises pellets providing an additional delayed release of methotrexate.

According to an embodiment, the invention concerns the pharmaceutical composition according to the invention, wherein each pellet has a size of less than 5.00 mm, 4.75 mm, 4.50 mm, 4.25 mm, 4.00 mm, 3.75 mm, 3.50 mm, 3.25 mm, 3.00 mm, 2.75 mm, 2.50 mm, 2.25 mm, 2.00 mm, 1.75 mm, 1.50 mm, 1.25 mm, 1.00 mm, 0.75 mm, 0.50 mm or less than 0. 25 mm.

According to an embodiment, the invention concerns the pharmaceutical composition according to the invention, wherein said pellets have a size selected among 0.01 - 5 mm, 0.1 - 4.5 mm, 0.2 - 4 mm, 0.3 - 3.5 mm, 0.4 - 3 mm, 0.5 - 2.5 mm, 0.6 - 2 mm, 0.75 - 1.5 mm, and about 1 mm.

According to an embodiment, the invention concerns the pharmaceutical composition according to the invention, wherein at least one of said pellets provides immediate release of methotrexate.

According to an embodiment, the invention concerns the pharmaceutical composition according to the invention comprising at least 15 mg methotrexate, wherein at least one of said pellets provides a predetermined delay of release of methotrexate.

According to an embodiment, the invention concerns the pharmaceutical composition according to the invention, wherein 0-40%, more preferred 5-35%, preferably 10-30%, more preferred 15-25%, preferably about 20% of the total amount of methotrexate has been released after 1 hour.

According to an embodiment, the invention concerns the pharmaceutical composition according to the invention, wherein 20-80%, more preferred 25-70%, preferably 30-60%, more preferred 35-50%, preferably about 40% of the total amount of methotrexate has been released after 2 hours.

According to an embodiment, the invention concerns the pharmaceutical composition according to the invention, wherein 30-100%, more preferred 40-90%, preferably 50-80%, more preferred 60-70%, preferably about 60% of the total amount of methotrexate has been released after 3 hours.

According to an embodiment, the invention concerns the pharmaceutical composition according to the invention, wherein 40-100%, more preferred 50-95%, preferably 60-90%, more preferred 70-85%, preferably about 80% of the total amount of methotrexate has been released after 4 hours.

According to an embodiment, the invention concerns the pharmaceutical composition according to the invention, wherein the upper limit of release is valid for the total contents of the pharmaceutical composition selected among at least 10, 15, 20, 25 and 30 mg methotrexate.

According to an embodiment, the invention concerns the pharmaceutical composition according to the invention, wherein at least 80 % of the total amount of methotrexate has been released within 4 hours of administration.

According to an embodiment, the invention concerns the pharmaceutical composition according to the invention, wherein said at least one pellet providing immediate release of methotrexate, allows release of at least 80% of the active ingredient of said pellet in 500 ml 0.1 N HCl, measured in a USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, or within 60 minutes.

According to an embodiment, the invention concerns the pharmaceutical composition according to the invention comprising at least 15 mg methotrexate, wherein said at least one pellet providing delayed release and/or a predetermined lag time prior to release of methotrexate, allows release of at least 80% of the active ingredient of said pellet in 500 ml 0.1 N HCl, measured in an USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes, 125 minutes, 130 minutes, 135 minutes, 140 minutes, 145 minutes, 150 minutes, 155 minutes, 160 minutes, 165 minutes, 170 minutes, 175 minutes, 180 minutes, 185 minutes, 190 minutes, 195 minutes, 200 minutes, 205 minutes, 210 minutes, 215 minutes, 220 minutes, 225 minutes, 230 minutes, 235 minutes or within 240 minutes.

According to an embodiment, the invention concerns the pharmaceutical composition according to the invention comprising at least 15 mg methotrexate, wherein said at least one pellet providing delayed release and/or a predetermined lag time prior to release of methotrexate, provides release of less than 20% of the active ingredient of said pellet in 500 ml 0.1 N HCl, measured in an USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes, 125 minutes, 130 minutes, 135 minutes, 140 minutes, 145 minutes, 150 minutes, 155 minutes, 160 minutes, 165 minutes, 170 minutes, 175 minutes, 180 minutes, 185 minutes, 190 minutes, 195 minutes, 200 minutes, 205 minutes, 210 minutes, 215 minutes, 220 minutes, 225 minutes, 230 minutes, 235 minutes or within 240 minutes.

According to an embodiment, the invention concerns the pharmaceutical composition according to the invention, wherein said composition allows release of at least 80% of the active ingredient in 500 ml 0.1 N HCl, measured in an USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes, 125 minutes, 130 minutes, 135 minutes, 140 minutes, 145 minutes, 150 minutes, 155 minutes, 160 minutes, 165 minutes, 170 minutes, 175 minutes, 180 minutes, 185 minutes, 190 minutes, 195 minutes, 200 minutes, 205 minutes, 210 minutes, 215 minutes, 220 minutes, 225 minutes, 230 minutes, 235 minutes or within 240 minutes. HCI may be referred to as hydrochloric acid.

A dissolution experiment evaluated the rate and extent that a compound dissolves to form a solution under carefully controlled conditions.

According to an embodiment, the invention concerns the pharmaceutical composition according to the invention, wherein said composition allows release of at least 80% of the active ingredient in 500 ml phosphate buffer pH 6.8, measured in a USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes, 125 minutes, 130 minutes, 135 minutes, 140 minutes, 145 minutes, 150 minutes, 155 minutes, 160 minutes, 165 minutes, 170 minutes, 175 minutes, 180 minutes, 185 minutes, 190 minutes, 195 minutes, 200 minutes, 205 minutes, 210 minutes, 215 minutes, 220 minutes, 225 minutes, 230 minutes, 235 minutes or within 240 minutes.

According to an embodiment, the invention concerns the pharmaceutical composition according to the invention, wherein said composition allows release of at least 80% of the active ingredient in 0.9% saline, measured in a USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes, 125 minutes, 130 minutes, 135 minutes, 140 minutes, 145 minutes, 150 minutes, 155 minutes, 160 minutes, 165 minutes, 170 minutes, 175 minutes, 180 minutes, 185 minutes, 190 minutes, 195 minutes, 200 minutes, 205 minutes, 210 minutes, 215 minutes, 220 minutes, 225 minutes, 230 minutes, 235 minutes or within 240 minutes.

According to an embodiment, the invention concerns the pharmaceutical composition according to the invention comprising at least 15 mg methotrexate, wherein at least one of the pellets comprises at least one excipient and/or coating controlling the release of said methotrexate.

According to an embodiment, the invention concerns the pharmaceutical composition, wherein at least one of the pellets comprises a coating controlling the release of methotrexate, wherein said coating comprises at least two excipients:
a. A first coating excipient; and
b. A second coating excipient, wherein said second coating excipient preferably has higher solubility than said first coating excipient; preferably said solubility is measured in an aqueous solvent, such as selected among water, 0.1 N HCl, phosphate buffer pH 6.8, and 0.9% saline.

According to an embodiment, the invention concerns the pharmaceutical composition, wherein said first coating excipient is selected among ethyl cellulose, shellac, cellulose acetate, Eudragit RL (acrylic polymer), Eudragit RS, Eudragit NE (acrylcopolymer), Kollicoat SR 30 D (poly vinyl acetate), and a mixture of any of these.

According to an embodiment, the invention concerns the pharmaceutical composition, wherein said second coating excipient is selected among hypromellose, methylcellulose, Polyethylene glycol (6000), Eudragit L, hypromellose acetate succinate, polyvinyl alcohol, and a mixture of any of these.

According to an embodiment, the invention concerns the pharmaceutical composition, wherein said first coating excipient comprises ethyl cellulose and/or said second coating excipient comprises hypromellose.

According to an embodiment, the invention concerns the pharmaceutical composition, wherein said coating comprises at least 15%, 14%, 13%, 12%, 11%, 10%, 9%, 8%, 7%, 6%, 5%, 4.5%, 4%, 3.5%, 3%, 2.9%, 2.8%, 2.7%, 2.6%, 2.5%, 2.4%, 2.3%, 2.2%, 2.1%, 2%, 1.9%, 1.8%, 1.7%, 1.6%, 1.5%, 1.4%, 1.3%, 1.2%, 1.1%, 1%, 0.9%, 0.8%, 0.7%, 0.6%, 0.5%, 0.4%, 0.3%, 0.2% or at least 0.1% w/w of said first coating excipient and/or said second coating excipient.

According to an embodiment, the invention concerns the pharmaceutical composition, wherein said coating comprises more than 15%, 14%, 13%, 12%, 11%, 10%, 9%, 8%, 7%, 6%, 5%, 4.5%, 4%, 3.5%, 3%, 2.9%, 2.8%, 2.7%, 2.6%, 2.5%, 2.4%, 2.3%, 2.2%, 2.1%, 2%, 1.9%, 1.8%, 1.7%, 1.6%, 1.5%, 1.4%, 1.3%, 1.2%, 1.1%, 1%, 0.9%, 0.8%, 0.7%, 0.6%, 0.5%, 0.4%, 0.3%, 0.2% or at least 0.1% w/w of said first coating excipient and/or said second coating excipient.

According to an embodiment, the invention concerns the pharmaceutical composition, wherein said first plurality of pellets comprises an amount selected among 2 - 20, 4 - 15, 6 - 10, and about 8 % w/w of said first coating excipient and/or said second coating excipient. According to an embodiment, the invention concerns the pharmaceutical composition, wherein said second plurality of pellets comprises an amount selected among 0.1 - 15, 0.25 - 10, 0.5 - 5, 1 - 4, 1.5 - 3, and about 2 % w/w of said first coating excipient and/or said second coating excipient.

According to an embodiment, the invention concerns the pharmaceutical composition, wherein said third plurality of pellets comprises about an amount selected among 0.1 - 15, 0.25 - 10, 0.5 - 5, 1 - 4, 1.5 - 3, and about 1.8 % w/w of said first coating excipient and/or said second coating excipient.

According to an embodiment, the invention concerns the pharmaceutical composition, wherein at least one of the pellets comprises a core, such as a tablet, wherein said core comprises:
i. methotrexate or a pharmaceutically acceptable salt thereof;
ii. at least one binder and/or filler;
iii. at least one disintegrant and/or dissolution excipient; and
iv. preferably at least one lubricant glidant.

According to an embodiment, the invention concerns the pharmaceutical composition, wherein said at least one binder and/or filler is selected among the group consisting of saccharides and their derivatives, such as disaccharides (sucrose, lactose); polysaccharides and their derivatives, such as starches, cellulose or modified cellulose, such as microcrystalline cellulose, and cellulose ethers, such as hydroxypropyl cellulose (HPC); sugar alcohols such as xylitol, sorbitol or mannitol; protein such as gelatin; synthetic polymers, such as polyvinylpyrrolidone (PVP) and polyethylene glycol (PEG); and a mixture of any of these.

Preferably at least one excipient has suitable binding properties, and at least one excipient has suitable filling properties. It may be the same excipient which has both suitable binding and filling properties.

According to a preferred embodiment, at least one first excipient has suitable binding properties, while at least one second excipient has suitable filling properties.

According to an embodiment, the invention concerns the pharmaceutical composition, wherein said at least one binder and/or filler is selected among the group consisting of microcrystalline cellulose, lactose monohydrate, and a mixture of these.

According to an embodiment, the invention concerns the pharmaceutical composition, wherein said at least one disintegrant and/or dissolution excipient is selected among the group consisting of sodium starch glycolate, crosslinked polymers, such as crosslinked polyvinylpyrrolidone (crospovidone) or crosslinked sodium carboxymethyl cellulose (croscarmellose sodium), and a mixture of any of these.

According to an embodiment, the invention concerns the pharmaceutical composition, wherein said at least one disintegrant and/or dissolution excipient is sodium starch glycolate.

According to an embodiment, the invention concerns the pharmaceutical composition, wherein said at least one lubricant is selected among the group consisting of talc, silica, and fats, such as vegetable stearin, magnesium stearate or stearic acid, and a mixture of any of these.

According to an embodiment, the invention concerns the pharmaceutical composition, wherein said at least one lubricant is magnesium stearate.

According to an embodiment, the invention concerns the pharmaceutical composition, wherein the total amount of binder and filler is selected among 10 - 90, 20 - 80, 30 - 70, 40 - 60, 50 - 55, and about 52 % w/w.

According to an embodiment, the invention concerns the pharmaceutical composition, wherein the total amount of disintegrant and dissolution excipient is selected among 5- 95, 10 - 90, 15 - 80, 20 - 70, 25 - 60, 30 - 50, 35 - 45 and about 40 % w/w.

According to an embodiment, the invention concerns the pharmaceutical composition, wherein the total amount of lubricant is selected among 0.1 - 5, 0.2 - 3, 0.3 - 2, 0.5 - 1.5 and about 1 % w/w.

According to an embodiment, the invention concerns the pharmaceutical composition, wherein said excipient and/or coating controlling the release of said methotrexate is ethyl cellulose and/or Hypromellose. Hypromellose may be referred to as HPMC.

According to an embodiment, the invention concerns the pharmaceutical composition according to the invention, wherein said excipient and/or coating controlling the release of said methotrexate is ethyl cellulose added Hypromellose.

According to an embodiment, the invention concerns the pharmaceutical composition according to the invention, wherein said coating comprises at least 15%, 14%, 13%, 12%, 11%, 10%, 9%, 8%, 7%, 6%, 5%, 4.5%, 4%, 3.5%, 3%, 2.9%, 2.8%, 2.7%, 2.6%, 2.5%, 2.4%, 2.3%, 2.2%, 2.1%, 2%, 1.9%, 1.8%, 1.7%, 1.6%, 1.5%, 1.4%, 1.3%, 1.2%, 1.1%, 1%, 0.9%, 0.8%, 0.7%, 0.6%, 0.5%, 0.4%, 0.3%, 0.2% or at least 0.1% Hypromellose.

According to an embodiment, the invention concerns the pharmaceutical composition according to the invention, wherein said coating comprises more than 15%, 14%, 13%, 12%, 11%, 10%, 9%, 8%, 7%, 6%, 5%, 4.5%, 4%, 3.5%, 3%, 2.9%, 2.8%, 2.7%, 2.6%, 2.5%, 2.4%, 2.3%, 2.2%, 2.1%, 2%, 1.9%, 1.8%, 1.7%, 1.6%, 1.5%, 1.4%, 1.3%, 1.2%, 1.1%, 1%, 0.9%, 0.8%, 0.7%, 0.6%, 0.5%, 0.4%, 0.3%, 0.2% or at least 0.1% Hypromellose.

According to an embodiment, the invention concerns the pharmaceutical composition according to the invention, wherein said first plurality of pellets comprises about 8 % Hypromellose (This may be a Pellet A).

According to an embodiment, the invention concerns the pharmaceutical composition according to the invention, wherein said second plurality of pellets comprises about 2 % Hypromellose (This may be a Pellet B).

According to an embodiment, the invention concerns the pharmaceutical composition according to the invention, wherein said third plurality of pellets comprises about 1.8 % Hypromellose (This may be a Pellet C).

According to an embodiment, the invention concerns the pharmaceutical composition according to the invention, wherein said pharmaceutical composition is for oral administration.

According to an embodiment, the invention concerns the pharmaceutical composition according to the invention, wherein at least 81%, 82%, 83%, 84%, 85%, 86%, 87%, 88%, 89%, 90%, 91%, 92%, 93%, 94%, 95%, 96%, 97%, 98%, 99% or at least 100% of the total methotrexate have been released within 4 hours.

According to an embodiment, the invention concerns the pharmaceutical composition according to the invention, wherein said pellet comprises 0.1 mg - 5 mg methotrexate, 0.15 mg - 2 mg, 0.2 mg - 1.5 mg, 0.25 mg - 1 mg, 0.3 mg - 0.8 mg, 0.35 mg - 0.6 mg, 0.4 mg - 0.5 mg, 0.45 mg - 0.5.5 m or about 0.45 methotrexate.

According to an embodiment, the invention concerns the pharmaceutical composition according to the invention, wherein said composition comprises at least 2.5 mg, 5 mg, 7.5 mg, 10 mg, 12.5 mg, 15 mg, 17.5 mg, 20 mg, 22.5 mg, 25 mg, 27.5 mg or at least 30 mg methotrexate.

According to an embodiment, the invention concerns the pharmaceutical composition according to the invention, wherein said composition comprises between 1 mg - 50 mg methotrexate, between 2 mg - 49 mg, 3 mg - 48 mg, 4 mg - 47 mg, 5 mg - 46 mg, 6 mg - 45 mg, 7 mg - 44mg, 8 mg - 43 mg, 9 mg - 42 mg, 10 mg - 41 mg, 11 mg - 40 mg, 12 mg - 39 mg, 13 mg - 38 mg, 14 mg - 37 mg, 15 mg - 36 mg, 16 mg - 35 mg, 17 mg - 34 mg, 18 mg - 33 mg, 19 mg - 32 mg, 20 mg - 31 mg, 21 mg - 30 mg, 22 mg - 29 mg, 23 mg - 28 mg, 24 mg - 27, 25 - 26 mg or about 25 mg methotrexate.

According to an embodiment, the invention concerns the pharmaceutical composition according to the invention, wherein a plurality of pellets providing immediate release comprises a total of at least 2.5 mg, 5 mg, 7.5 mg, 10 mg, 12.5 mg, 15 mg, 17.5 mg, 20 mg, 22.5 mg, 25 mg, 27.5 mg or a total of at least 30 mg methotrexate.

According to an embodiment, the invention concerns the pharmaceutical composition according to the invention, wherein said composition is for use in a method of treatment of a disease and/or for use in a prophylaxis of a disease.

According to an embodiment, the invention concerns the pharmaceutical composition according to the invention, wherein said disease is an arthritis.

According to an embodiment, the invention concerns the pharmaceutical composition according to the invention, wherein said arthritis is selected among Rheumatoid arthritis, juvenile idiopathic arthritis (and subsets thereof, such as Polyarticular) and or psoriasis arthritis.

According to an embodiment, the invention concerns the pharmaceutical composition according to the invention, wherein said disease is selected among psoriasis, Crohn's disease and or a cancer.

According to an embodiment, the invention concerns the pharmaceutical composition according to the invention, wherein said cancer is selected among a breast cancer, a cancer vesicae urinariae, choriocarcinoma, a head and neck cancer, a lymphoma and a leukemia.

According to an embodiment, the invention concerns the pharmaceutical composition according to the invention, wherein the pellets have color depending on their release characteristics.

Using different colors for different pellets depending on the release characteristics make it possible to make quality check by visual inspection after the pellets have been mixed together to form the pharmaceutical composition. Further, patients may be able to verify the dosage, and different colors may increase patient compliance, in particular among younger patients such as children and for their parents assisting them in taking the treatment.

According to an embodiment, the invention concerns the pharmaceutical composition according to the invention, wherein each of said pellets is a tablet.

According to an embodiment, the invention concerns the pharmaceutical composition according to the invention, wherein a plurality of said pellets are compressed to form a tablet.

According to an embodiment, the invention concerns the pharmaceutical composition, wherein said tablet is adapted to disintegrate in the stomach and/or small intestine of a subject.

According to an embodiment, the invention concerns the pharmaceutical composition according to the invention, wherein said pellets are in a capsule or a sachet.

According to an embodiment, the invention concerns the pharmaceutical composition according to the invention, wherein said composition comprises or is obtainable by combining a number of pellets selected among 1-200, 1-150, 1-120, 1-100, 1-90, 1-80, 1-70, 1-66, 1-60, 2-50, 3-40, 4-30, 5-25, 6-20, 7-18, 8-16, 9-14, 10-12, and 11 pellets.

According to an embodiment, the invention concerns the pharmaceutical composition according to the invention, wherein said composition is administered once a week, or twice within 12, preferably 8 hours, once a week.

According to an embodiment, the invention concerns a method for the manufacture of a pharmaceutical composition according to the invention.

According to an embodiment, the invention concerns a method for the manufacture of a pharmaceutical composition according to the invention comprising:
i. Manufacturing at least two different types of pellets, wherein each type of pellet has specific release characteristics, and
ii. Mixing the pellets according to the desired final release characteristics and total amount of methotrexate.

According to an embodiment, the invention concerns the method or pharmaceutical formulation according to the invention, wherein methotrexate is replaced or accompanied with one or more active ingredients, preferably wherein at least one active ingredient is a chemotherapeutic agent.

According to an embodiment, the invention concerns the use of methotrexate or a pharmaceutically acceptable salt thereof for the manufacture of a medicament for the treatment of a disease, such as a pharmaceutical composition according to the invention.

According to an embodiment, the invention concerns a method of treating the disease according to the invention, comprising administering a therapeutically effective amount of a composition of the invention.

### Figures

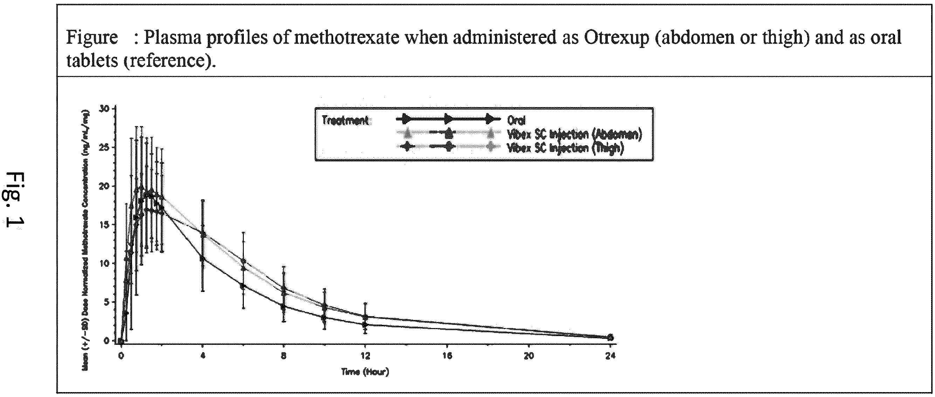

Fig. 1 shows concentration-time profiles of MTX following PO (Peroral) administration of 15 mg MTX and concentration-time profiles of MTX following SC (subcutaneous) administration of 15 mg MTX.
Fig. 2 shows release profiles from IR (immediate release) mini-tablets in 0.1 N HCL.
Fig. 3 shows release profiles from IR (immediate release) mini-tablets in phosphate buffer pH 6.8.
Fig. 4 shows release from minitablets coated with 25% HPMC film.
Fig. 5 shows release from minitablets coated with 22.5% HPMC film.

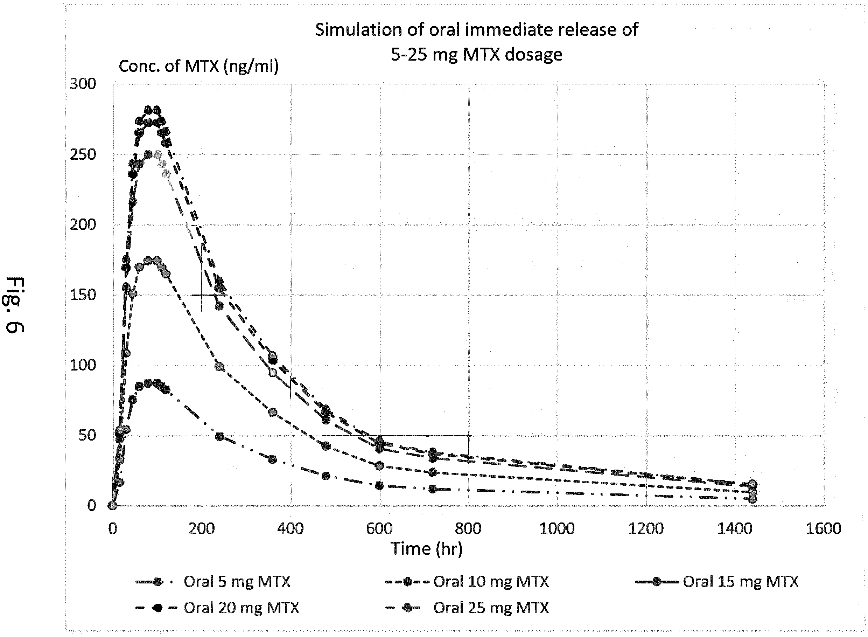

Fig. 6 shows simulation of pharmacokinetic profiles of oral immediate release dosing 5-25 mg (ng/ml^{∗}min).

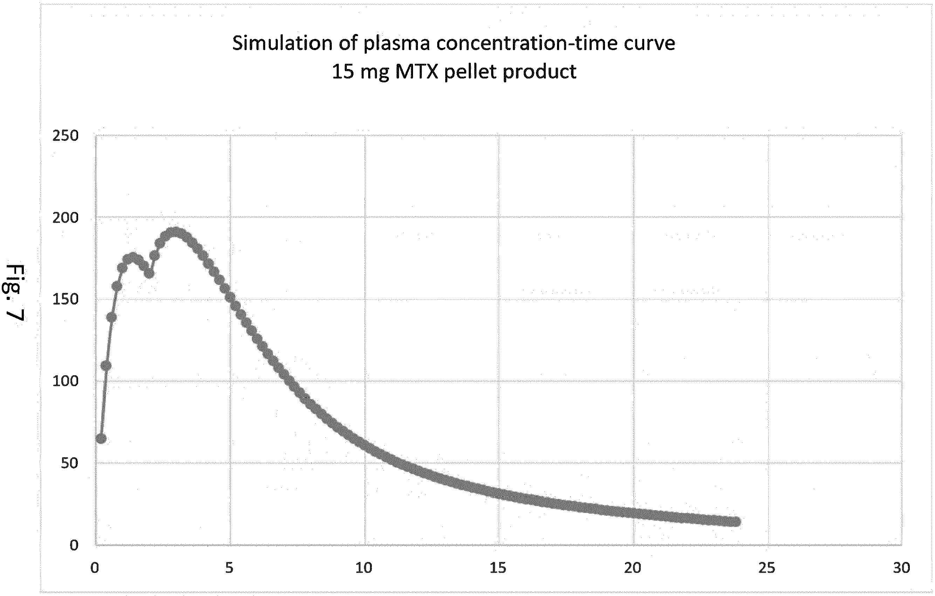

Fig. 7 shows the pharmacokinetic profile of calculated result of two doses of MTX given two hours apart. 10 mg fast release and 5 mg slow release: Ka for first dose is 1.48 (immediate release) and Ka for the second dose is 0.7. AUC (0-24h) = 1711 ng/mL^{∗}hr.

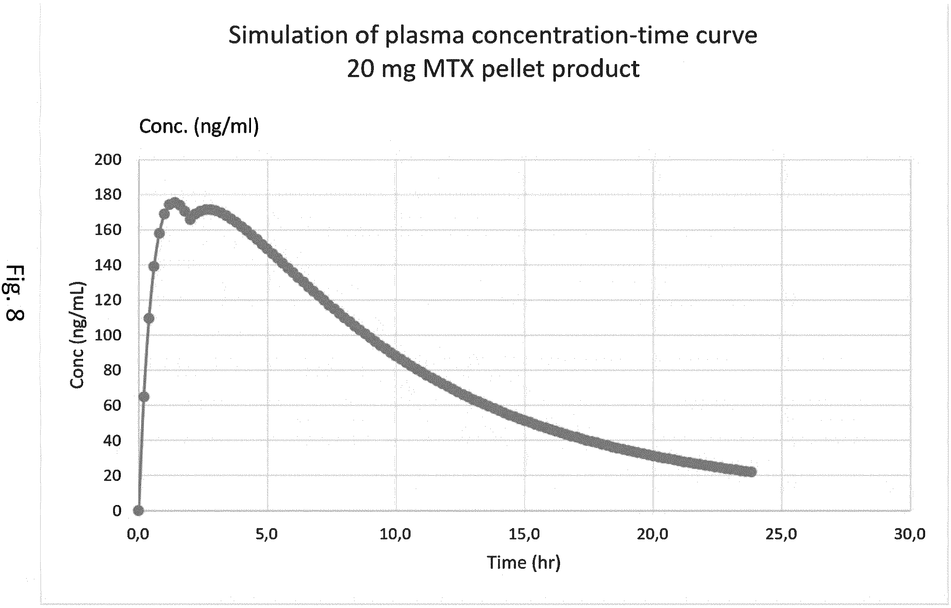

Fig. 8 shows the pharmacokinetic profile of calculated result of two doses given two hours apart: 10 mg fast release and 10 mg slow release: Ka for first dose is 1.48 (immediate release) and Ka for the second dose is 0.22. F= 80% for the second dose. AUC(0-24h) = 2000 ng/mL^{∗}hr.

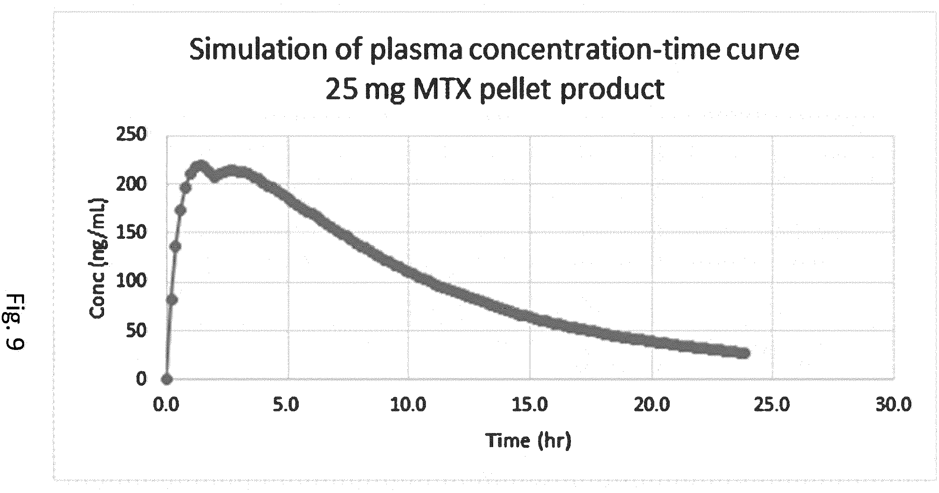

Fig. 9 shows the pharmacokinetic profile of calculated result of two doses given two hours apart: 12.5 mg fast release and 12.5 mg slow release: Ka for first dose is 1.48 (immediate release) and Ka for the second dose is 0.22. AUC(0-24h)=2657 ng/mL^{∗}hr. Cmax is at the point of "saturation level".

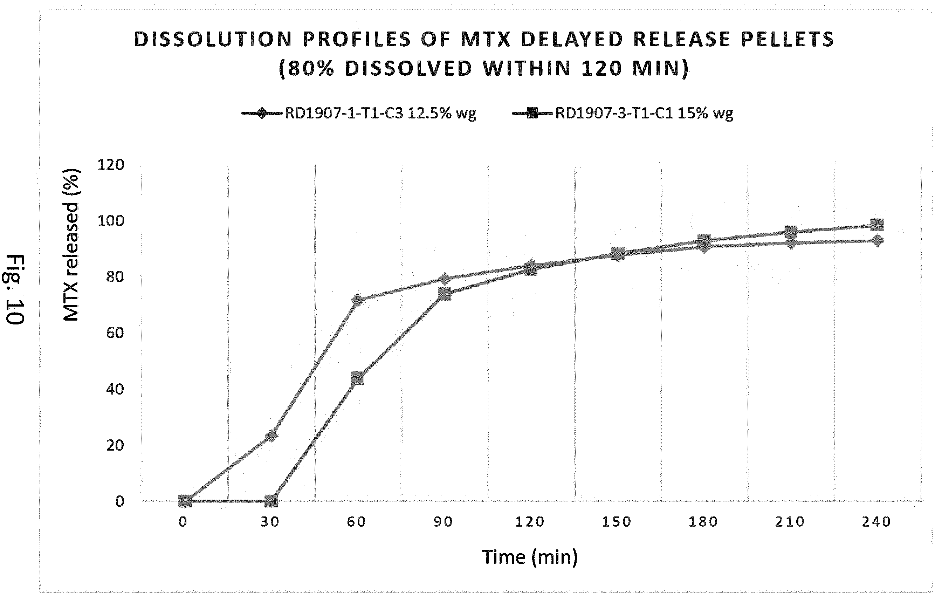

Fig. 10 shows dissolution profile of delayed release minitablets (80% released within 120 minutes).

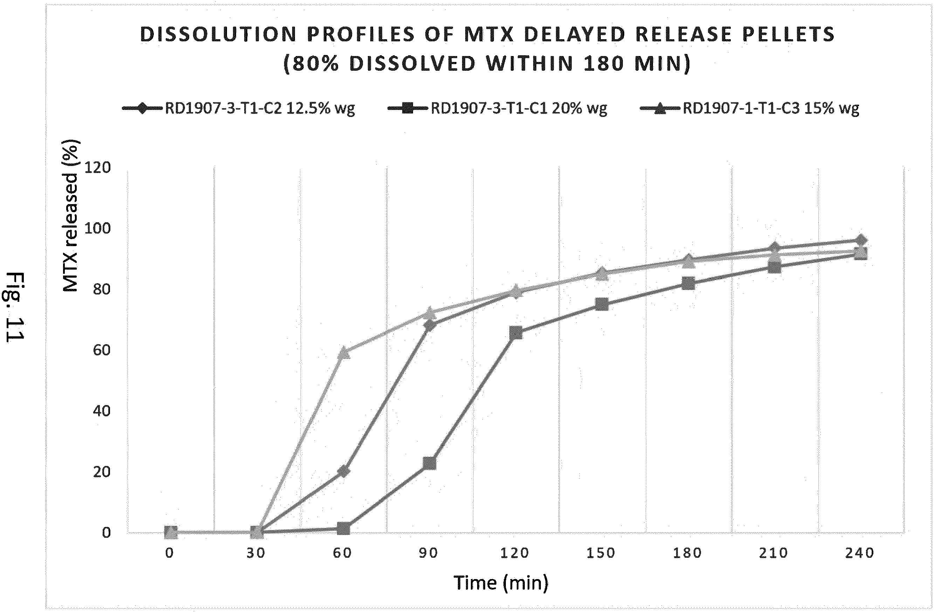

Fig. 11 shows dissolution profiles of delayed release minitablets (80% released within 180 minutes).

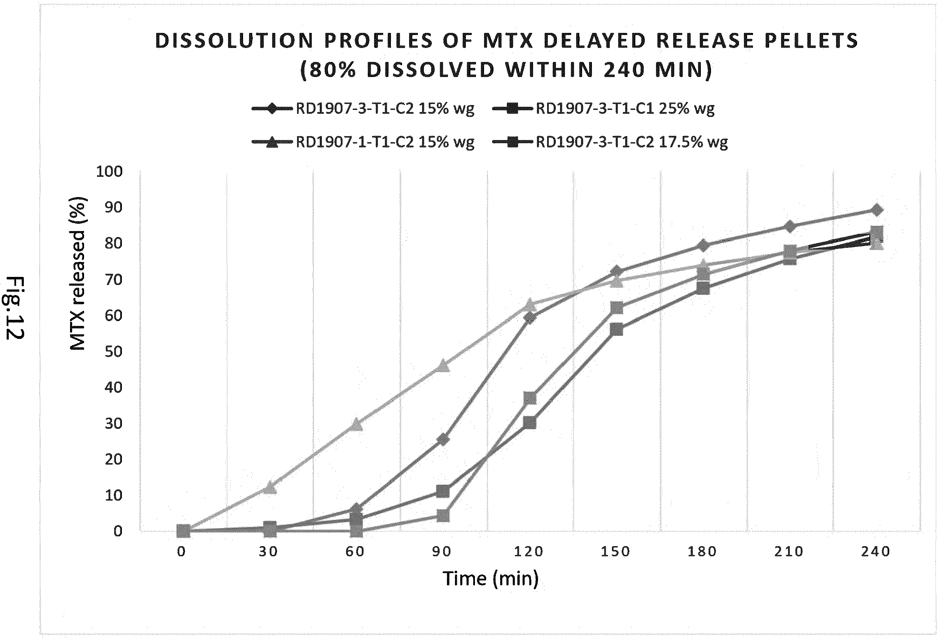

Fig. 12 shows dissolution profiles of delayed release minitablets (80% released within 240 minutes).

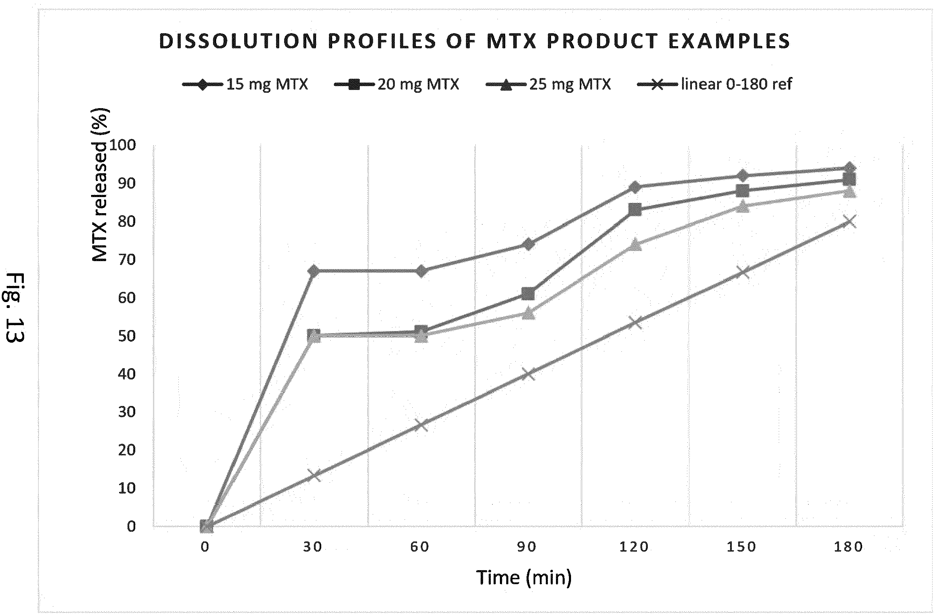

Fig. 13 shows simulations of dissolution profiles of preferred embodiments of the invention. The straight line shows what the profile would look like if the release was linear.

All cited references are incorporated by reference.

The accompanying Figures and Examples are provided to explain rather than limit the present invention. It will be clear to the person skilled in the art that aspects, embodiments, claims and any items of the present invention may be combined.

Unless otherwise mentioned, all percentages are in weight/weight. Unless otherwise mentioned, all measurements are conducted under standard conditions (ambient temperature and pressure). Unless otherwise mentioned, test conditions are according to European Pharmacopoeia 8.0.

Certain aspects and embodiments of the present invention are expressed in the following items:
1. A pharmaceutical composition comprising a plurality of pellets, wherein each pellet has a smallest diameter ≤ 5 mm, preferably ≤ 3 mm, and wherein each pellet comprises methotrexate or a pharmaceutically acceptable salt thereof.
2. A pharmaceutical composition comprising a plurality of pellets, wherein each pellet comprises methotrexate or a pharmaceutically acceptable salt thereof,
   and preferably wherein at least one of the pellets provides immediate release of said methotrexate;
   and preferably wherein at least one of the pellets provides delayed release and/or a predetermined lag time prior to release of methotrexate;
   and wherein at least 80 % of the total amount of methotrexate of said pharmaceutical composition have been released within 4 hours of administration and/or as measured in an USP Dissolution Apparatus 2 in 500 ml 0.1 N HCI at 37°C ± 0.5°C.
3. A pharmaceutical composition comprising
   i. At least one pellet providing immediate release of methotrexate or a pharmaceutically acceptable salt thereof, and
   ii. If the pharmaceutical composition comprises more than 10 mg methotrexate further at least one pellet providing delayed release of methotrexate;
   Wherein said pharmaceutical composition allows release of at least 80 % of the total amount of methotrexate within 4 hours of administration.
4. A pharmaceutical composition comprising a plurality of pellets, said composition comprising a total dosage of 1 - 30 mg methotrexate or a pharmaceutically acceptable salt thereof, and wherein said composition comprises:
   a. At least one pellet A providing immediate release of methotrexate.
   b. For the composition comprising more than 10 mg, such as at least 12.5 mg, more preferred 15 mg, preferably 17.5 mg, more preferred 20 mg methotrexate, further at least one pellet B providing delayed release of methotrexate, and preferably
   c. For the composition comprising at least 15 mg, preferably 17.5 mg, more preferred 20 mg methotrexate, further at least one pellet C providing delayed release of methotrexate wherein said pellet C provides slower release than said pellet B.
5. A pharmaceutical composition comprising a plurality of at least two, preferably at least three, pellets, providing a total dosage of methotrexate or a pharmaceutically acceptable salt thereof of 1 - 30 mg, wherein said composition comprises pellets providing immediate release of methotrexate;
   and for dosages of 12.5 - 30 mg further comprises pellets providing delayed release of methotrexate;
   and for dosages of 20 - 30 mg comprises pellets providing an additional delayed release of methotrexate.
6. A pharmaceutical composition comprising a total amount of methotrexate, or a pharmaceutically acceptable salt thereof, of 15 - 50, preferably 20-30 mg, wherein said pharmaceutical composition releases at least 80% of the total amount of methotrexate within 4 hours; and
   wherein said pharmaceutical composition comprises
   i) at least one immediate release pharmaceutical composition comprising 10 - 15 mg methotrexate and providing release of at least 8 - 10 mg of methotrexate within 1 hour; and
   ii) at least one delayed release pharmaceutical composition comprising a content of methotrexate of at least 2.5 mg, more preferred at least 5 mg methotrexate,
   wherein said delayed release pharmaceutical composition releases less than 50% of said content of methotrexate within 1 hour; preferably each composition comprises one or more pellets, such as one or more tablets.
7. A delayed release pharmaceutical composition comprising a content of methotrexate, or a pharmaceutically acceptable salt thereof, of at least 2.5 mg, more preferred at least 5 mg methotrexate, wherein said delayed release pharmaceutical composition releases less than 50% of said content of methotrexate within 1 hour;
   for use in a treatment comprising oral administration of a total amount of methotrexate, or a pharmaceutically acceptable salt thereof, of 15 - 50, preferably 20-30 mg;
   wherein said treatment further comprises administration of at least one immediate release pharmaceutical composition comprising 10 - 15 mg methotrexate and providing release of at least 8 - 10 mg of methotrexate within 1 hour,
   subject to the proviso than said delayed release pharmaceutical dosage forms and said immediate release pharmaceutical composition releases at least 80% of the total amount of methotrexate within 4 hours;
   preferably each composition comprises one or more pellets, such as one or more tablets.
8. An immediate release pharmaceutical composition comprising 10 - 15 mg methotrexate, or a pharmaceutically acceptable salt thereof, and providing release of at least 8 - 10 mg of methotrexate within 1 hour,
   for use in a treatment comprising oral administration of a total amount of methotrexate, or a pharmaceutically acceptable salt thereof, of 15 - 50, preferably 20-30 mg;
   wherein said treatment further comprises administration of at least one delayed release pharmaceutical composition comprising a content of methotrexate, or a pharmaceutically acceptable salt thereof, of at least 2.5 mg, more preferred at least 5 mg methotrexate, wherein said delayed release pharmaceutical composition releases less than 50% of said content of methotrexate within 1 hour;
   subject to the proviso than said delayed release pharmaceutical dosage forms and said immediate release pharmaceutical composition releases at least 80% of the total amount of methotrexate within 4 hours;
   preferably each composition comprises one or more pellets, such as one or more tablets.
9. The pharmaceutical composition according to any of the preceding items, wherein each pellet has a size of less than 5.00 mm, 4.75 mm, 4.50 mm, 4.25 mm, 4.00 mm, 3.75 mm, 3.50 mm, 3.25 mm, 3.00 mm, 2.75 mm, 2.50 mm, 2.25 mm, 2.00 mm, 1.75 mm, 1.50 mm, 1.25 mm, 1.00 mm, 0.75 mm, 0.50 mm or less than 0. 25 mm.
10. The pharmaceutical composition according to any of the preceding items, wherein said pellets have a size selected among 0.01 - 5 mm, 0.1 - 4.5 mm, 0.2 - 4 mm, 0.3 - 3.5 mm, 0.4 - 3 mm, 0.5 - 2.5 mm, 0.6 - 2 mm, 0.75 - 1.5 mm, and about 1 mm.
11. The pharmaceutical composition according to any of the preceding items, wherein at least one of said pellets provides immediate release of methotrexate.
12. The pharmaceutical composition according to any of the preceding items comprising at least 10 mg, preferably at least 12.5 mg, more preferred at least 15 mg methotrexate, wherein at least one of said pellets provides a predetermined delay of release of methotrexate.
13. The pharmaceutical composition according to any of the preceding items, wherein 20-90%, more preferred 30-80%, preferably 40-70%, more preferred at least 50% of the total amount of methotrexate has been released after 1 hour.
14. The pharmaceutical composition according to any of the preceding items, wherein 50-100%, more preferred 60-95%, preferably 65-90%, more preferred at least 70% of the total amount of methotrexate has been released after 2 hours.
15. The pharmaceutical composition according to any of the preceding items, wherein 60-100%, more preferred 70-99%, preferably 80-98%, more preferred more than 85% of the total amount of methotrexate has been released after 3 hours.
16. The pharmaceutical composition according to any of the preceding items, wherein 80-100%, more preferred 85-99%, preferably 90-98%, more preferred 93-97.5% of the total amount of methotrexate has been released after 4 hours.
17. The pharmaceutical composition according to any of items 13 - 16, comprising an amount of methotrexate selected among at least 10, 12.5, 15, 20, 25 and 30 mg.
18. The pharmaceutical composition according to any of the preceding items, wherein at least 80 % of the total amount of methotrexate has been released within 4 hours, preferably 3 hours, of administration.
19. The pharmaceutical composition according to any of the preceding items, wherein said at least one pellet providing immediate release of methotrexate, allows release of at least 80% of the active ingredient of said pellet in in 500 ml 0.1 N HCl, measured in a USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, or within 60 minutes.
20. The pharmaceutical composition according to any of the preceding items, wherein said at least one pellet providing delayed release and/or a predetermined lag time prior to release of methotrexate, allows release of at least 80% of the active ingredient of said pellet in in 500 ml 0.1 N HCl, measured in an USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes, 125 minutes, 130 minutes, 135 minutes, 140 minutes, 145 minutes, 150 minutes, 155 minutes, 160 minutes, 165 minutes, 170 minutes, 175 minutes, 180 minutes, 185 minutes, 190 minutes, 195 minutes, 200 minutes, 205 minutes, 210 minutes, 215 minutes, 220 minutes, 225 minutes, 230 minutes, 235 minutes or within 240 minutes.
21. The pharmaceutical composition according to any of the preceding items, wherein said at least one pellet providing delayed release and/or a predetermined lag time prior to release of methotrexate, provides release of at least 80% of the active ingredient of said pellet in in 500 ml 0.1 N HCl, measured in an USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes, 125 minutes, 130 minutes, 135 minutes, 140 minutes, 145 minutes, 150 minutes, 155 minutes, 160 minutes, 165 minutes, 170 minutes, 175 minutes, 180 minutes, 185 minutes, 190 minutes, 195 minutes, 200 minutes, 205 minutes, 210 minutes, 215 minutes, 220 minutes, 225 minutes, 230 minutes, 235 minutes or within 240 minutes.
22. The pharmaceutical composition according to any of the preceding items, wherein said composition allows release of at least 80% of the active ingredient in in 500 ml 0.1 N HCl, measured in an USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes, 125 minutes, 130 minutes, 135 minutes, 140 minutes, 145 minutes, 150 minutes, 155 minutes, 160 minutes, 165 minutes, 170 minutes, 175 minutes, 180 minutes, 185 minutes, 190 minutes, 195 minutes, 200 minutes, 205 minutes, 210 minutes, 215 minutes, 220 minutes, 225 minutes, 230 minutes, 235 minutes or within 240 minutes.
23. The pharmaceutical composition according to any of the preceding items, wherein said composition allows release of at least 80% of the active ingredient in 500 ml phosphate buffer pH 6.8, measured in an USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes, 125 minutes, 130 minutes, 135 minutes, 140 minutes, 145 minutes, 150 minutes, 155 minutes, 160 minutes, 165 minutes, 170 minutes, 175 minutes, 180 minutes, 185 minutes, 190 minutes, 195 minutes, 200 minutes, 205 minutes, 210 minutes, 215 minutes, 220 minutes, 225 minutes, 230 minutes, 235 minutes or within 240 minutes.
24. The pharmaceutical composition according to any of the preceding items, wherein said composition allows release of at least 80% of the active ingredient in 0.9% saline, measured in an USP Dissolution Apparatus 2, Paddle, (37°C ± 0.5°C), within 5 minutes, 10 minutes, 15 minutes, 20 minutes, 25 minutes, 30 minutes, 35 minutes, 40 minutes, 45 minutes, 50 minutes, 55 minutes, 60 minutes, 65 minutes, 70 minutes, 75 minutes, 80 minutes, 85 minutes, 90 minutes, 95 minutes, 100 minutes, 105 minutes, 110 minutes, 115 minutes, 120 minutes, 125 minutes, 130 minutes, 135 minutes, 140 minutes, 145 minutes, 150 minutes, 155 minutes, 160 minutes, 165 minutes, 170 minutes, 175 minutes, 180 minutes, 185 minutes, 190 minutes, 195 minutes, 200 minutes, 205 minutes, 210 minutes, 215 minutes, 220 minutes, 225 minutes, 230 minutes, 235 minutes or within 240 minutes.
25. The pharmaceutical composition according to any of the preceding items, wherein at least one of the pellets comprises at least one excipient and/or coating controlling the release of methotrexate.
26. The pharmaceutical composition according to any of the preceding items, wherein at least one of the pellets comprises a coating controlling the release of methotrexate, wherein said coating comprises at least two excipients:
   a. A first coating excipient; and
   b. A second coating excipient, wherein said second coating excipient preferably has higher solubility than said first coating excipient; preferably said solubility is measured in an aqueous solvent, such as selected among water, 0.1 N HCl, phosphate buffer pH 6.8, and 0.9% saline.
27. The pharmaceutical composition according to item 26, wherein said first coating excipient is selected among ethyl cellulose, shellac, cellulose acetate, Eudragit RL (acrylic polymer), Eudragit RS, Eudragit NE (acrylcopolymer), Kollicoat SR 30 D (poly vinyl acetate), and a mixture of any of these.
28. The pharmaceutical composition according to item 26 or 27, wherein said second coating excipient is selected among hypromellose, methylcellulose, Polyethylene glycol (6000), Eudragit L, hypromellose acetate succinate, polyvinyl alcohol, and a mixture of any of these.
29. The pharmaceutical composition according to any of the items 26 - 28, wherein said first coating excipient comprises ethyl cellulose and/or said second coating excipient comprises hypromellose.
30. The pharmaceutical composition according to any of the preceding items, wherein said coating comprises at least 15%, 14%, 13%, 12%, 11%, 10%, 9%, 8%, 7%, 6%, 5%, 4.5%, 4%, 3.5%, 3%, 2.9%, 2.8%, 2.7%, 2.6%, 2.5%, 2.4%, 2.3%, 2.2%, 2.1%, 2%, 1.9%, 1.8%, 1.7%, 1.6%, 1.5%, 1.4%, 1.3%, 1.2%, 1.1%, 1%, 0.9%, 0.8%, 0.7%, 0.6%, 0.5%, 0.4%, 0.3%, 0.2% or at least 0.1% w/w of said first coating excipient and/or said second coating excipient.
31. The pharmaceutical composition according to any of the preceding items, wherein said coating comprises more than 15%, 14%, 13%, 12%, 11%, 10%, 9%, 8%, 7%, 6%, 5%, 4.5%, 4%, 3.5%, 3%, 2.9%, 2.8%, 2.7%, 2.6%, 2.5%, 2.4%, 2.3%, 2.2%, 2.1%, 2%, 1.9%, 1.8%, 1.7%, 1.6%, 1.5%, 1.4%, 1.3%, 1.2%, 1.1%, 1%, 0.9%, 0.8%, 0.7%, 0.6%, 0.5%, 0.4%, 0.3%, 0.2% or at least 0.1% w/w of said first coating excipient and/or said second coating excipient.
32. The pharmaceutical composition according to any of the preceding items, wherein said first plurality of pellets comprises an amount selected among 2 - 20, 4 - 15, 6 - 10, and about 8 % w/w of said first coating excipient and/or said second coating excipient.
33. The pharmaceutical composition according to any of the preceding items, wherein said second plurality of pellets comprises an amount selected among 0.1 - 15, 0.25 - 10, 0.5 - 5, 1 - 4, 1.5 - 3, and about 2 % w/w of said first coating excipient and/or said second coating excipient.
34. The pharmaceutical composition according to any of the preceding items, wherein said third plurality of pellets comprises about an amount selected among 0.1 - 15, 0.25 - 0.5 - 5,1 - 4, 1.5 - 3, and about 1.8 % w/w of said first coating excipient and/or said second coating excipient.
35. The pharmaceutical composition according to any of the preceding items, wherein at least one of the pellets comprises a core, such as a tablet, wherein said core comprises:
   i. methotrexate or a pharmaceutically acceptable salt thereof;
   ii. at least one binder and/or filler;
   iii. at least one disintegrant and/or dissolution excipient; and
   iv. preferably at least one lubricant glidant.
36. The pharmaceutical composition according to item 35, wherein said at least one binder and/or filler is selected among the group consisting of saccharides and their derivatives, such as disaccharides (sucrose, lactose); polysaccharides and their derivatives, such as starches, cellulose or modified cellulose, such as microcrystalline cellulose, and cellulose ethers, such as hydroxypropyl cellulose (HPC); sugar alcohols such as xylitol, sorbitol or mannitol; protein such as gelatin; synthetic polymers, such as polyvinylpyrrolidone (PVP) and polyethylene glycol (PEG); and a mixture of any of these.
37. The pharmaceutical composition according to item 35 or 36, wherein said at least one binder and/or filler is selected among the group consisting of microcrystalline cellulose, lactose monohydrate, and a mixture of these.
38. The pharmaceutical composition according to any of the items 35 - 37, wherein said at least one disintegrant and/or dissolution excipient is selected among the group consisting of sodium starch glycolate, crosslinked polymers, such as crosslinked polyvinylpyrrolidone (crospovidone) or crosslinked sodium carboxymethyl cellulose (croscarmellose sodium), and a mixture of any of these.
39. The pharmaceutical composition according to any of the items 35 - 38, wherein said at least one disintegrant and/or dissolution excipient is sodium starch glycolate.
40. The pharmaceutical composition according to any of the items 35 - 39, wherein said at least one lubricant is selected among the group consisting of talc, silica, and fats, such as vegetable stearin, magnesium stearate or stearic acid, and a mixture of any of these.
41. The pharmaceutical composition according to any of the items 35 - 40, wherein said at least one lubricant is magnesium stearate.
42. The pharmaceutical composition according to any of the items 35 - 41, wherein the total amount of binder and filler is selected among 10 - 90, 20 - 80, 30 - 70, 40 - 60, 50 - 55, and about 52 % w/w.
43. The pharmaceutical composition according to any of the items 35 - 42, wherein the total amount of disintegrant and dissolution excipient is selected among 5- 95,10 - 90, 15 - 80, 20 - 70, 25 - 60, 30 - 50, 35 - 45 and about 40 % w/w.
44. The pharmaceutical composition according to any of the items 35 - 43, wherein the total amount of lubricant is selected among 0.1 - 5, 0.2 - 3, 0.3 - 2, 0.5 - 1.5 and about 1 % w/w.
45. The pharmaceutical composition according to any of the preceding items, wherein said pharmaceutical composition is for oral administration.
46. The pharmaceutical composition according to any of the preceding items, wherein at least 81%, 82%, 83%, 84%, 85%, 86%, 87%, 88%, 89%, 90%, 91%, 92%, 93%, 94%, 95%, 96%, 97%, 98%, 99% or at least 100% of the total methotrexate have been released within 4 hours.
47. The pharmaceutical composition according to any of the preceding items, wherein said pellet comprises 0.1 mg - 5 mg methotrexate, 0.15 mg - 2 mg, 0.2 mg - 1.5 mg, 0.25 mg - 1 mg, 0.3 mg - 0.8 mg, 0.35 mg - 0.6 mg, 0.4 mg - 0.5 mg, 0.45 mg - 0.5.5 m or about 0.45 mg methotrexate.
48. The pharmaceutical composition according to any of the preceding items, wherein said composition comprises at least 2.5 mg, 5 mg, 7.5 mg, 10 mg, 12.5 mg, 15 mg, 17.5 mg, 20 mg, 22.5 mg, 25 mg, 27.5 mg or at least 30 mg methotrexate.
49. The pharmaceutical composition according to any of the preceding items, wherein said composition comprises between 1 mg-50 mg methotrexate, between 2 mg-49 mg, 3 mg - 48 mg, 4 mg - 47 mg, 5 mg - 46 mg, 6 mg - 45 mg, 7 mg - 44mg, 8 mg - 43 mg, 9 mg - 42 mg, 10 mg - 41 mg, 11 mg - 40 mg, 12 mg - 39 mg, 13 mg - 38 mg, 14 mg - 37 mg, 15 mg - 36 mg, 16 mg - 35 mg, 17 mg - 34 mg, 18 mg - 33 mg, 19 mg - 32 mg, 20 mg - 31 mg, 21 mg - 30 mg, 22 mg - 29 mg, 23 mg - 28 mg, 24 mg - 27, 25 - 26 mg or about 25 mg methotrexate.
50. The pharmaceutical composition according to any of the preceding items, wherein the plurality of pellets providing immediate release comprises a total of no more than 2.5 mg, 5 mg, 7.5 mg, 10 mg, 12.5 mg, 15 mg methotrexate.
51. The pharmaceutical composition according to any of the preceding items, wherein said composition is for use in a method of treatment of a disease and/or for use in a prophylaxis of a disease and/or for alleviating the symptoms of a disease.
52. The pharmaceutical composition according to any of the preceding items, wherein said disease is an arthritis.
53. The pharmaceutical composition according to any of the preceding items, wherein said arthritis is selected among Rheumatoid arthritis, polyarticular juvenile idiopathic arthritis and or psoriasis arthritis.
54. The pharmaceutical composition according to any of the preceding items, wherein said disease is selected among psoriasis, Crohn's disease and or a cancer.
55. The pharmaceutical composition according to any of the preceding items, wherein said cancer is selected among a breast cancer, a cancer vesicae urinariae, choriocarcinoma, a head and neck cancer, a lymphoma and a leukemia.
56. The pharmaceutical composition according to any of the preceding items, for use in a treatment, wherein the subject is a child or less than 18 years old.
57. The pharmaceutical composition according to any of the preceding items, wherein the pellets have colours depending on their release characteristics.
58. The pharmaceutical composition according to any of the preceding items, wherein each of said pellets is a tablet.
59. The pharmaceutical composition according to any of the preceding items, wherein a plurality of said pellets are compressed to form a tablet.
60. The pharmaceutical composition according to item 59, wherein said tablet is adapted to disintegrate in the stomach of a subject.
61. The pharmaceutical composition according to any of the preceding items, wherein said pellets are in a capsule or a sachet.
62. The pharmaceutical composition according to any of the preceding items, wherein said composition comprises or is obtainable by combining a number of pellets selected among 1-200, 1-150, 1-120, 1-100, 1-90, 1-80, 1-70, 1-66, 1-60, 2-50, 3-40, 4-30, 5-25, 6-20, 7-18, 8-16, 9-14, 10-12, and 11 pellets.
63. The pharmaceutical composition according to any of the preceding items, for use in a treatment wherein said composition is administered once a week or twice within 12, preferably 8 hours once a week.
64. A method for the manufacture of a pharmaceutical composition according to any of the preceding items.
65. A method for the manufacture of a pharmaceutical composition comprising more than 10 mg methotrexate according to any of the preceding items comprising:
   i. Manufacturing at least two different types of pellets, wherein each type of pellet has specific release characteristics, and
   ii. Mixing the pellets according to the desired final release characteristics and total amount of methotrexate.
66. The method or pharmaceutical formulation according to any of the preceding items, wherein methotrexate is replaced or accompanied with one or more active ingredients, preferably wherein at least one active ingredient is a chemotherapeutic agent.
67. Use of the pharmaceutical composition according to any of the preceding items in the manufacturing of a medicament for the treatment of a disease, wherein said disease is a disease according to any of the preceding items.
68. A method of treating the disease according to any of the preceding items, comprising administering a therapeutically effective amount of the composition of any of the preceding items.
69. Method of oral administration of a dosage form comprising a total amount of methotrexate, or a pharmaceutically acceptable salt thereof, of 15 - 50, preferably 20-30 mg,
   wherein said dosage form comprises
   i) at least one immediate release pharmaceutical composition comprising 10 - 15 mg methotrexate and providing release of at least 8 - 10 mg of methotrexate within 1 hour; and
   ii) at least one delayed release pharmaceutical composition comprising a content of methotrexate of at least 2.5 mg, more preferred at least 5 mg methotrexate,
   wherein said delayed release pharmaceutical composition releases less than 50% of said content of methotrexate within 1 hour;
   subject to the proviso that said dosage form releases at least 80% of the total amount of methotrexate within 4 hours;
   preferably each composition comprises one or more pellets, such as one or more tablets.

### Examples

### Development of methotrexate mini tablets

Mini-tablet formulations are developed with immediate release and with lag time/burst release to deliver the dose in the intestine within maximum 4 hours from dosing. These different formulations can then be combined in a capsule and may increase bioavailability compared to conventional tablets.

Substances: Methotrexate disodium, Microcrystalline cellulose, Lactose monohydrate, Lactose Monohydrate (spraydried), Sodium starch glycolate, Magnesium stearate, Ethycellulose 7 cps, Hypromellose 3 cps, Ethanol 96%, Purified water.

Methotrexate exists as either the acid (molecular weight: 454.4) or the di-sodium salt (molecular weight: 498.40). In pharmaceutical products, Methotrexate is declared as the acid, but often added as the di-sodium salt (conversion factor 1.096), as this is freely soluble in water; the acid is practically insoluble in water.

Methotrexate di-sodium salt was sourced from Fermion, Finland.

Placebo mini-tablets for initial film coating experiments were produced in-house and were composed by Lactose monohydrate (67%), Microcrystalline cellulose (27%), Croscarmellose sodium (5%) and Magnesium stearate (1%) and compressed at a tooling similar to the size used for the Methotrexate mini-tablets.

Major equipment used is listed in table 1

**Table 1 - equipment**

| Process | **Equipment** | **Type and size** |
|---|---|---|
| Mixing | Blender | Turbula blender |
| Compression | Tablet press | Diaf TM20 single punch tablet press with 2.0 mm round tooling |
| Film coating | Fliud-bed with wurster | Aeromatic-Fielder STREA-1 |

### Methods

The aim is a dose of 15 mg Methotrexate given with two or three release profiles, the first profile being immediate release (IR). Later profiles must deliver the dose within three-four hours post dosing.

With up to three release profiles, each profile can hold 5 mg dose. This was formulated in 11 mini-tablets each with tablet weight of 7.0 mg to be contained in a small capsule size (3^{∗}11 mini-tablets).

Excipients were chosen from those used in marketed products (e.g. Emthexate (TEVA) containing Microcrystalline cellulose, starch, magnesium stearate, silicon dioxide and lactose monohydrate).

### Methotrexate dissolution

Methotrexate dissolution was tested using the following parameters:
USP dissolution <711>, apparatus 2
Dissolution medium: 0.1 N HCI
500 mL dissolution medium, 75 RPM
Sample times: every half hour up to 6 hours.
Samples were quantified using HPLC or UV absorption at 303 nm.

### Results and Discussion

### Laboratory experiments

First mixing experiments were a test of whether direct compression was possible or a granulation had to be introduced. Formulations were based on earlier experience with similar formulation principle (Table 2). In the second formulation Microcrystalline cellulose had been exchanged with Lactose monohydrate, otherwise formulations were identical.

The ingredients except magnesium stearate were mixed in the Turbula mixer at 22 rpm for 5 minutes in a 350 mL container. Magnesium stearate was added, and mixed in for 1 minute.

**Table 2 Composition of core tablets**

| Batch no | RD1907-1-T1 | | RD1907-2-T1 | |
|---|---|---|---|---|
| Methotrexate disodium | 2.847 g | 7.117% | 1.423 g | 7.117% |
| Microcrystalline cellulose | 8.000 g | 20.0% | - | - |
| Lactose monohydrate | 12.753 g | 31.833% | - | - |
| Lactose Monohydrate spray dried | - | - | 10.377 g | 51.883% |
| Sodium starch glycolate Type A | 16.000 g | 40.0% | 8.000 g | 40.000% |
| Magnesium stearate | 0.400 g | 1.00% | 0.200 g | 1.000% |
| Total | 40.0 g | - | 20.0 g | - |

Mini-tablets were compressed on the Diaf single punch tablet press using 2 mm tooling. In-process control was tablet weight (40 mini-tablets) and tablet thickness and friability (1 g product tested for 5 minutes at 45 rpm in Turbula mixer). The mix's compressed well; data are presented in Table 3.

A third batch, RD1907-3-T1, was compressed with same composition as RD1907-1-T1 to have enough mini-tablets for coating experiments.

**Table 3: In-process data from compression**

| Batch no | RD1907-1-T1 | RD1907-2-T1 | RD1907-3-T1 |
|---|---|---|---|
| Strength | 5 mg / 11 mini-tablets | | |
| Compression setting | 3 1/6 | | |
| Compression speed | Approx. 65 stroke/min | | 55 stroke/min |
| Tablet weight / RSD | 7.011 mg / 1.95% | 7.537 mg / 0.70% | 6.971 mg / 0.96% |
| Tablet thickness | 1.84 mm | 1.87 mm | 1.81 mm |
| Friability | 0.46% | 1.00% | 0.77% |

Active mini-tablets were mixed with placebo mini-tablets to allow film coating in the STREA fluid-bed. As the active tablets were yellow from the API, mini-tablets could easily be sorted after coating. Typically, 10 g active mini-tablets were used; coating batch size was 300 g.

To achieve an IR formulation, mini-tablets were coated with a HPMC film (Table 4). Different barrier films based on ethylcellulose added hypromellose to form pores in the film, were produced and coated onto mini-tablets to achieve delayed burst release. By decreasing the content of hypromellose in the film, the film improved in delaying release.

**Table 4: Composition of film coating**

| Film | HPMC film | | 20% HPMC | | 22.5% HPMC | | 25% HPMC | |
|---|---|---|---|---|---|---|---|---|
| Ethocel 7 cps | - | - | 44.8 g | 6.4% | 55.8 g | 6.2% | 42.0 g | 6.0% |
| Ethanol 96% | 448.5 g | 69.0% | 483.0 g | 69.0% | 621.0 g | 69.0% | 483.0 g | 69.0% |
| Hypromellose 3 | 52.0 g | 8.0% | 11.2 g | 1.6% | 16.2 g | 1.8% | 14.0 g | 2.0% |
| Purified water | 149.5 g | 23.0% | 161.0 g | 23.0% | 207.0 g | 23.0% | 161.0 g | 23.0% |
| Total | 650 g | - | 700 g | - | 900 g | - | 700 g | - |

Table 5 gives standard process parameters for film coating in STREA fluid-bed. At specified weight gains, the fluid-bed was stopped and samples drawn to be tested for dissolution.

**Table 5: Process parameters for film coating**

| | |
|---|---|
| Batch size (tablets) | 300 g |
| Atomizer air (bar) | 0.75-1.0 |
| Inlet air temperature (°C) | Approx. 32 |
| Outlet air temperature (°C) | 26-28 |
| PV flow rate (g/min) | Approx. 4.0 |

### IR product

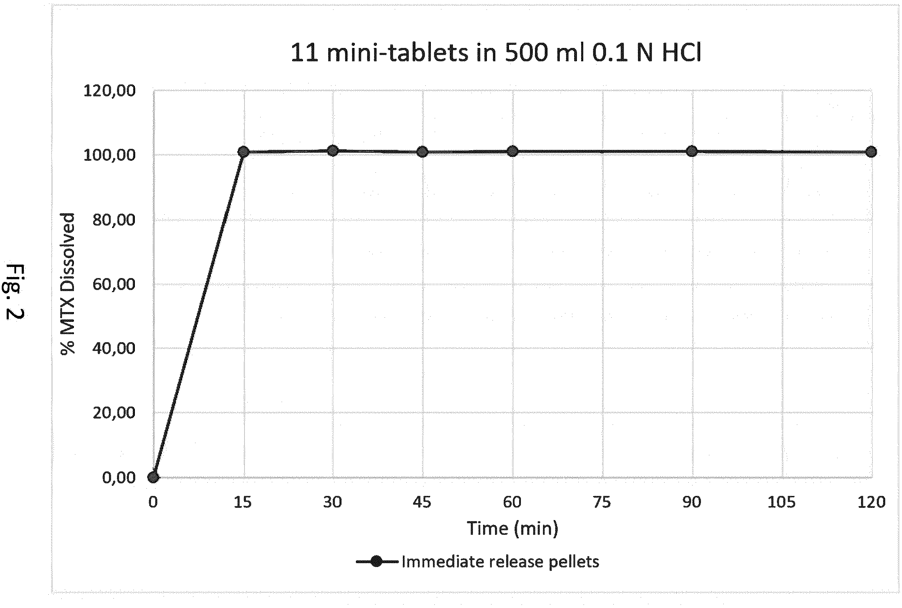

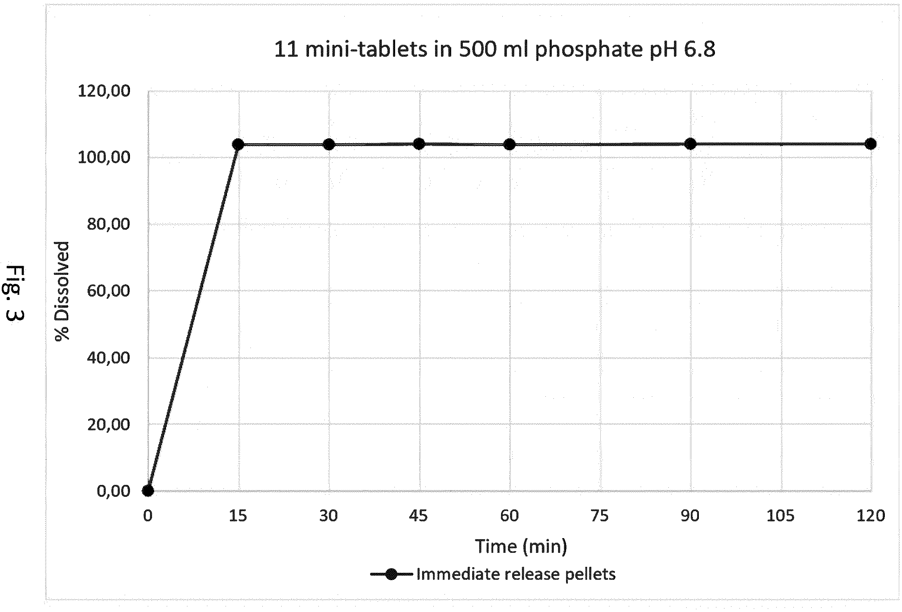

Batch RD1907-1-T1 was coated with the HPMC film as batch RD1907-1-T1-C1. The coating process was stopped at 15% weight gain, and the mini-tablets were tested in dissolution in three media: 0.1 N HCl, phosphate buffer pH 6.8 and 0.9% saline. 11 mini-tablets corresponding to 5 mg methotrexate were tested in 500 mL media at 75 rpm. Results are presented in Figure 2 and Figure 3, which demonstrate that Methotrexate were release within 15 minutes independent on media used.

### Delayed release products

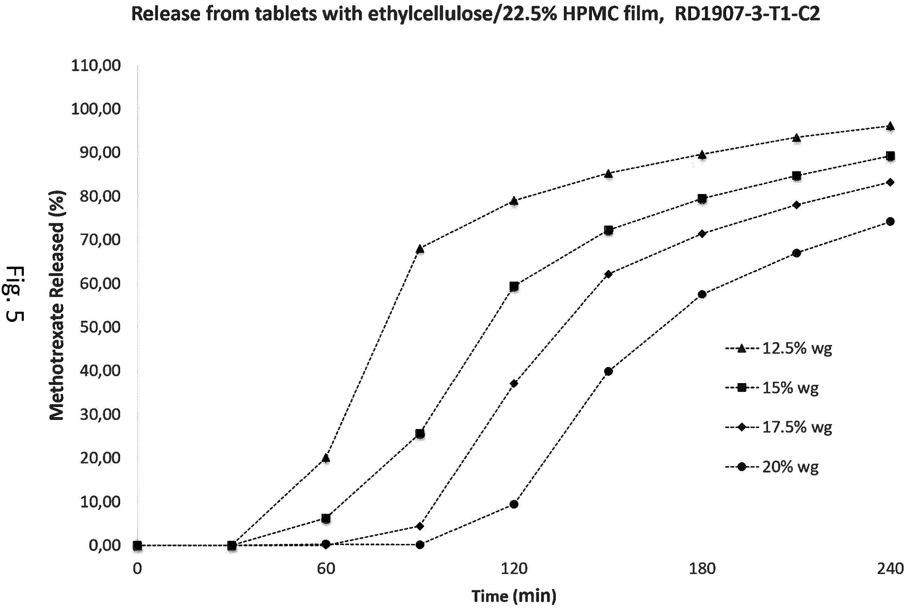

Batch RD1907-1-T1 and RD1907-2-T1 were coated with the 20% HPMC film as batch RD1907-1-T1-C2 and RD1907-2-T1-C1 respectively, with sampling after 15%, 17.5% and 20% weight gain (Figure 5). The release rate was found to be too slow when release was achieved without achieving a delay before release was started, and none of the batches released fully (> 80%) within 4 hours. Further the batch RD1907-2-T1-C1 released slower and more incomplete compared to batch RD1907-1-T1-C2 which must be caused by the core tablet holding different filler. This core composition is not optimal for this formulation.

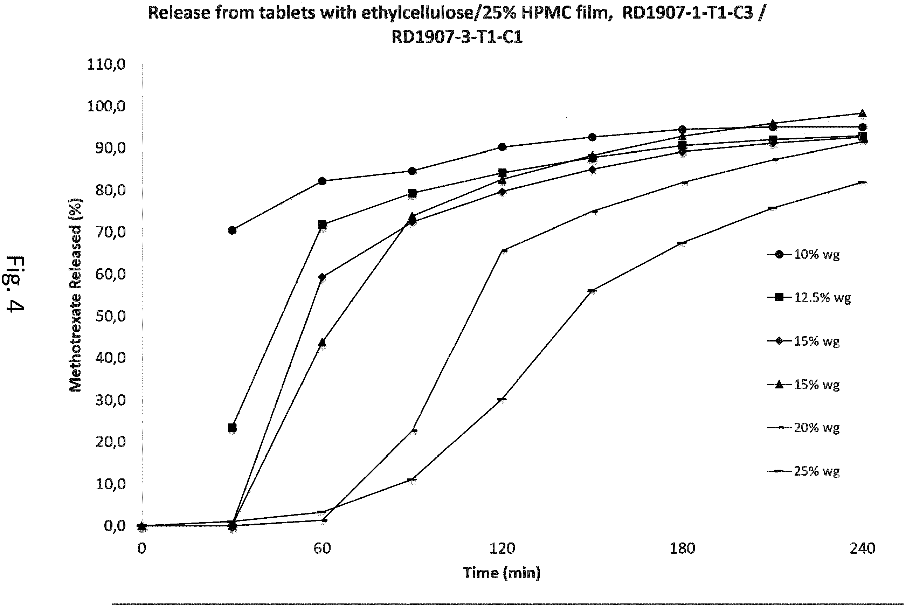

To achieve steeper release and a lag time, Batch RD1907-1-T1 and RD1907-3-T1 were coated with first 25% HPMC film (Figure 4) and next 22.5% HPMC film (Figure 5).

With the 25% HPMC film, a lag time with no release was achieved with 15% weight gain or more. The release when first started was faster than for the 20% HPMC film, but decreased with thickness of the film/weight gain.

With 20% weight gain, a lag time of 1-1½ hour was achieved and 80% of the dose was released after 3 hours. However, release rate from 2 hours onwards was low (10% every 30 min).

Very similar results were achieved with the 22.5% HPMC film but with lower weight gain. 15% weight gain with this film gave same result as 20% weight gain with the 25% HPMC film. With the 22.5% HPMC film it was possible to achieve longer lag-time and still have a acceptable release rate.

### Conclusion

Mini-tablets with different release profiles all releasing the Methotrexate between 15 minutes and 4 hours have been developed. From these formulations, two or three release profiles can be selected to deliver the dose at intervals over the stomach and intestine.

A combination of two equal doses of immediate release MTX (ka =1,48) and slow release MTX (simulated to be released (ka = 0,22) at two hours after fist dose) seems to give high exposure of MTX. The second dose is expected to have a lower bioavailability of 80% in comparison to the first dose, due to less effective absorption from the lower part of the small intestine. A release that give rise to a plasma concentration lower than approximately 200 ng/ml is believed to be in the linear range of absorption from the gut of MTX. A combination of mini-tablets with different in-vitro release profiles may result in better bioavailability mimicking the pharmacokinetic profiles of subcutaneous dosing of MTX.

The experiments and calculations indicate an immediate release profile in combination with RD1907-3-T1-C1 20% as well as RD1907-3-T1-C2 17.5% WG is believed to offer the best combination of mini-tablets.

**Table 6 Release data**

| Time (min) / % released | 0 | 15 | 30 | 45 | 60 | 90 | 120 |
|---|---|---|---|---|---|---|---|
| RD1907-1-T1-C1 in 0.1N HCL | 0.00 | 103.5 | 103.9 | 103.7 | 103.8 | 104.0 | 103.9 |
| RD1907-1-T1-C1 in ph. 6.8 | 0.00 | 106.3 | 106.4 | 106.2 | 106.5 | 106.9 | 106.9 |
| RD1907-1-T1-C1 in 0.9% saline | 0.00 | 105.2 | 105.7 | 105.7 | 105.7 | 105.8 | 106.1 |

**Table 7a Release data**

| Time (min) / % released | 0 | 30 | 60 | 90 | 120 | 150 | 180 |
|---|---|---|---|---|---|---|---|
| RD1907-1-T1-C2 17.5% wg | 0.0 | 0.4 | 8.3 | 24.2 | 30.7 | 45.8 | 60.5 |
| RD1907-1-T1-C2 20% wg | 0.0 | 0.4 | 0.4 | 10.3 | 17.8 | 29.3 | 44.0 |
| RD1907-2-T1-C1 15% wg | 0.0 | 0.6 | 0.6 | 1.4 | 3.0 | 9.0 | 19.3 |
| RD1907-2-T1-C1 17.5% wg | 0.0 | 3.7 | 4.9 | 4.0 | 1.8 | 3.6 | 7.7 |
| RD1907-2-T1-C1 20% wg | 0.0 | 0.5 | 0.1 | 0.2 | 2.3 | 2.1 | 10.1 |
| RD1907-3-T1-C2 20% wg | 0.0 | 0.0 | 0.3 | 0.1 | 9.4 | 39.8 | 57.5 |
| Immediate release | 0.0 | 100 | 100 | 100 | 100 | 100 | 100 |
| RD1907-1-T1-C3 10% wg | 0.0 | 70.4 | 82.2 | 84.6 | 90.2 | 92.6 | 94.4 |
| RD1907-1-T1-C3 12.5% wg | 0.0 | 23.4 | 71.7 | 79.3 | 84.1 | 87.7 | 90.6 |
| RD1907-3-T1-C1 15% wg | 0.0 | 0.0 | 43.8 | 73.8 | 82.6 | 88.3 | 92.8 |
| RD1907-3-T1-C2 12.5% wg | 0.0 | 0.0 | 20.1 | 68.1 | 79.0 | 85.3 | 89.6 |
| RD1907-3-T1-C1 20% wg | 0.0 | 0.0 | 1.3 | 22.6 | 65.6 | 74.9 | 81.8 |
| RD1907-1-T1-C3 15% wg | 0.0 | 0.1 | 59.3 | 72.3 | 79.6 | 85.0 | 89.1 |
| RD1907-3-T1-C2 15% wg | 0.0 | 0.0 | 6.2 | 25.6 | 59.4 | 72.2 | 79.5 |
| RD1907-3-T1-C1 25% wg | 0.0 | 1.0 | 3.3 | 11.1 | 30.2 | 56.1 | 67.5 |
| RD1907-1-T1-C2 15% wg | 0.0 | 12.4 | 29.9 | 46.2 | 63.0 | 69.7 | 74.0 |
| RD1907-3-T1-C2 17.5% wg | 0.0 | 0.0 | 0.0 | 4.4 | 37.0 | 62.1 | 71.4 |

**Table 7b Release data**

| Time (min) / % released | 210 | 240 | 270 | 300 | 330 | 360 | number |
|---|---|---|---|---|---|---|---|
| RD1907-1-T1-C2 17.5% wg | 66.6 | 70.9 | 74.6 | 78.1 | - | - | |
| RD1907-1-T1-C2 20% wg | 53.8 | 61.2 | 66.4 | 70.7 | - | - | |
| RD1907-2-T1-C1 15% wg | 33.7 | 44.1 | 53.0 | 63.5 | - | - | |
| RD1907-2-T1-C1 17.5% wg | 19.0 | 31.4 | 43.2 | 51.4 | - | - | |
| RD1907-2-T1-C1 20% wg | 13.3 | 23.1 | 36.5 | 46.7 | - | - | |
| RD1907-3-T1-C2 20% wg | 67.0 | 74.2 | 80.3 | 85.1 | 89.7 | 92.3 | |
| Immediate release | 100 | 100 | 100 | 100 | | | 1 |
| RD1907-1-T1-C3 10% wg | 95.0 | 95.0 | 96.8 | 97.4 | - | - | 2 |
| RD1907-1-T1-C3 12.5% wg | 92.1 | 92.9 | 95.3 | 96.4 | - | - | 3 |
| RD1907-3-T1-C1 15% wg | 95.9 | 98.4 | 100.4 | 102.0 | 103.2 | 104.0 | 4 |
| RD1907-3-T1-C2 12.5% wg | 93.5 | 96.1 | 98.1 | 99.9 | 101.7 | 102.8 | 5 |
| RD1907-3-T1-C1 20% wg | 87.2 | 91.5 | 94.8 | 97.5 | 100.1 | 101.6 | 6 |
| RD1907-1-T1-C3 15% wg | 91.2 | 92.6 | 95.4 | 96.7 | - | - | 6B |
| RD1907-3-T1-C2 15% wg | 84.7 | 89.3 | 92.7 | 95.6 | 98.2 | 100.0 | 7 |
| RD1907-3-T1-C1 25% wg | 75.7 | 81.9 | 86.4 | 90.2 | 93.8 | 96.0 | 8 |
| RD1907-1-T1-C2 15% wg | 77.5 | 80.1 | 82.2 | 84.9 | - | - | 9 |
| RD1907-3-T1-C2 17.5% wg | 78.0 | 83.2 | 87.5 | 90.8 | 94.2 | 96.3 | 10 |

**Table 8a In-vitro dissolution**

| **mini-tablet profile number** | 1 | 2 | 3 | 4 | 5 |
|---|---|---|---|---|---|
| | IR product | RD1907-1-T1-C3 10% wg | RD1907-1-T1-C3 12.5% wg | RD1907-3-T1-C1 15% wg | RD1907-3-T1-C2 12.5% wg |
| 0 | 0 | 0 | 0 | 0 | 0 |
| 30 | 100 | 70,4 | 23,4 | 0 | 0 |
| 60 | 100 | 82,2 | 71,7 | 43,8 | 20,1 |
| 90 | 100 | 84,6 | 79,3 | 73,8 | 68,1 |
| 120 | 100 | 90,2 | 84,1 | 82,6 | 79 |
| 150 | 100 | 92,6 | 87,7 | 88,3 | 85,3 |
| 180 | 100 | 94,4 | 90,6 | 92,8 | 89,6 |
| 210 | 100 | 95 | 92,1 | 95,9 | 93,5 |
| 240 | 100 | 95 | 92,9 | 98,4 | 96,1 |
| % dissolved | At least 80% within one hour | At least 80% within one hour | At least 80% within two hours | At least 80% within two hours | At least 80% within three hours |

**Table 8b In-vitro dissolution**

| **mini-tablet profile number** | **6** | **6B** | **7** | **8** | **9** | **10** |
|---|---|---|---|---|---|---|
| | RD1907-3-T1-C1 20% wg | RD1907-1-T1-C3 15% wg | RD1907-3-T1-C2 15% wg | RD1907-3-T1-C1 25% wg | RD1907-1-T1-C2 15% wg | RD1907-3-T1-C2 17.5% wg |
| 0 | 0 | 0 | 0 | 0 | 0 | 0 |
| 30 | 0 | 0.1 | 0 | 1 | 12,4 | 0 |
| 60 | 1,3 | 59.3 | 6,2 | 3,3 | 29,9 | 0 |
| 90 | 22,6 | 72.3 | 25,6 | 11,1 | 46,2 | 4.4 |
| 120 | 65,6 | 79.6 | 59,4 | 30,2 | 63 | 37.0 |
| 150 | 74,9 | 85.0 | 72,2 | 56,1 | 69,7 | 62.1 |
| 180 | 81,8 | 89.1 | 79,5 | 67,5 | 74 | 71.4 |
| 210 | 87,2 | 91.2 | 84,7 | 75,7 | 77,5 | 78.0 |
| 240 | 91,5 | 92.6 | 89,3 | 81,9 | 80,1 | 83.2 |
| % dissolved | At least 80% within three hours | At least 80% within 3 hours | At least 80% within four hours | At least 80% within four hours | At least 80% within four hours | At least 80% within four hours |

**Table 9 Capsules with minitablets: Examples of Proposed Profile Combinations**

| **Profile** | **1** | **6** | **10** |
|---|---|---|---|
| 5 mg capsule | 5 mg | | |
| 7.5 mg capsule | 7.5 mg | | |
| 10 mg capsule | 10 mg | | |
| 12.5 mg capsule | 12.5 mg | | |
| 12.5 mg capsule | 10 mg | 2.5 mg | |
| 12.5 mg capsule | 7.5 mg | 5 mg | |
| 15 mg capsule | 12.5 mg | 2.5 mg | |
| 15 mg capsule | 10 mg | 5 mg | |
| 15 mg capsule | 7.5 mg | 7.5 mg | |
| 17.5 mg | 12.5 mg | 5 mg | |
| 17.5 mg capsule | 10 mg | 7.5 mg | |
| 20 mg capsule | 12.5 mg | 7.5 mg | |
| 20 mg capsule | 10 mg | 10 mg | |
| 22.5 mg capsule | 12.5 mg | 10 mg | |
| 22.5 mg capsules | 12.5 mg | 5 mg | 5 mg |
| 22.5 mg capsule | 10 mg | 10 mg | 2.5 mg |
| 22.5 mg capsule | 10 mg | 7.5 mg | 5 mg |
| 22.5 mg capsule | 10 mg | 5 mg | 7.5 mg |
| 25 mg capsule | 12.5 mg | 12.5 mg | |
| 25 mg capsule | 12.5 mg | 7.5 mg | 5 mg |
| 25 mg capsules | 12.5 mg | 5 mg | 7.5 mg |
| 25 mg capsule | 10 mg | 10 mg | 5 mg |
| 25 mg capsule | 10 mg | 7.5 mg | 7.5 mg |
| 25 mg capsule | 10 mg | 5 mg | 10 mg |
| 27.5 mg capsule | 12.5 mg | 10 mg | 5 mg |
| 27.5 mg capsule | 12.5 mg | 7.5 mg | 7.5 mg |
| 27.5 mg capsules | 12.5 mg | 5 mg | 10 mg |
| 27.5 mg capsule | 10 mg | 10 mg | 7.5 mg |
| 27.5 mg capsule | 10 mg | 7.5 mg | 10 mg |
| 30 mg capsule | 12.5 mg | 12.5 mg | 5 mg |
| 30 mg capsule | 12.5 mg | 10 mg | 7.5 mg |
| 30 mg capsule | 12.5 mg | 7.5 mg | 10 mg |
| 30 mg capsule | 10 mg | 10 mg | 10 mg |

Profile 1 may be substituted with profile 2, profile 6 may be substituted with profile 3, 4, 5 or 6B and profile 10 may be substituted with profile 7, 8 or 9.

**Table 10 Proposed combination of minitablets profiles for future manufacturing**

| Dose | Mini-tablet profile | Amount MTX | Mini-tablet profile | Amount MTX | n profiles |
|---|---|---|---|---|---|
| **5 mg** | Immediate release | 5 mg | | | 1 |
| **7.5 mg** | Immediate release | 7.5 mg | | | 1 |
| **10 mg** | Immediate release | 10 mg | | | 1 |
| **12.5 mg** | Immediate release | 12.5 mg | | | 1 |
| **15 mg** | Immediate release | 10 mg | RD-1907-3-T1-C1 20% WG | 5 mg | 2 |
| **17.5 mg** | Immediate release | 10 mg | RD-1907-3-T1-C1 20% WG | 7.5 mg | 2 |
| **20 mg** | Immediate release | 10 mg | RD-1907-3-T1-C1 20% WG | 5 mg | 3 |
| | | | RD1907-3-T1-C2 17.5% WG | 5 mg | |
| **22.5 mg** | Immediate release | 12.5 mg | RD-1907-3-T1-C1 20% WG | 5 mg | 3 |
| | | | RD1907-3-T1-C2 17.5% WG | 5 mg | |
| **25 mg** | Immediate release | 12.5 mg | RD-1907-3-T1-C1 20% WG | 5 mg | 3 |
| | | | RD1907-3-T1-C2 17.5% WG | 7.5 mg | |
| **30 mg** | Immediate release | 15 mg | RD-1907-3-T1-C1 20% WG | 7.5 mg | 3 |
| | | | RD1907-3-T1-C2 17.5% WG | 7.5 mg | |

The simulated and proposed combinations have been based on the following data and results:

**3 mini-tablet dissolution profiles**

| Time (min) / % released | 0 | 30 | 60 | 90 | 120 | 150 | 180 | 210 | 240 |
|---|---|---|---|---|---|---|---|---|---|
| immediate release | 0 | 100 | 100 | 100 | 100 | 100 | 100 | 100 | 100 |
| RD1907-3-T1-C1 20% wg | 0 | 0 | 1.3 | 22.6 | 65.6 | 74.9 | 81.8 | 87.2 | 91.5 |
| RD1907-3-T1-C2 17.5% wg | 0 | 0 | 0 | 4.4 | 37 | 62.1 | 71.4 | 78 | 83.2 |

**Table 11 Combination of mini-tablets: constructed dissolution profiles**

| Combination of mini-tablets: Constructed dissolution profiles | | | | | | | | | |
|---|---|---|---|---|---|---|---|---|---|
| | 0 | 30 | **60** | 90 | **120** | 150 | **180** | 210 | **240** |
| 15 mg composition | | | | | | | | | |
| 10mg | 0 | 66.7 | **66.7** | 66.7 | **66.7** | 66.7 | **66.7** | 66.7 | **66.7** |
| 5 mg | 0 | 0.0 | **0.4** | 7.5 | **21.9** | 25.0 | **27.3** | 29.1 | **30.5** |
| **Total** | **0** | **66.7** | **67.1** | **74.2** | **88.5** | **91.6** | **93.9** | **95.7** | **97.2** |

| 17.5 mg composition | | | | | | | | | |
|---|---|---|---|---|---|---|---|---|---|
| 10 mg | 0 | 57.1 | **57.1** | 57.1 | **57.1** | 57.1 | **57.1** | 57.1 | **57.1** |
| 7.5 mg | 0 | 0.0 | **0.6** | 9.7 | **28.1** | 32.1 | **35.1** | 37.4 | **39.2** |
| **Total** | **0** | **57.1** | **57.7** | **66.8** | **85.3** | **89.2** | **92.2** | **94.5** | **96.4** |

| 20 mg composition | | | | | | | | | |
|---|---|---|---|---|---|---|---|---|---|
| 10 mg | 0 | 50.0 | **50.0** | 50.0 | **50.0** | 50.0 | **50.0** | 50.0 | **50.0** |
| 10 mg | 0 | 0.0 | **0.7** | 11.3 | **32.8** | 37.5 | **40.9** | 43.6 | **45.8** |
| **Total** | **0** | **50.0** | **50.7** | **61.3** | **82.8** | **87.5** | **90.9** | **93.6** | **95.8** |

| 22.5 mg composition | | | | | | | | | |
|---|---|---|---|---|---|---|---|---|---|
| 12.5mg | 0 | 55.6 | **55.6** | 55.6 | **55.6** | 55.6 | **55.6** | 55.6 | **55.6** |
| 5 mg | 0 | 0.0 | **0.3** | 5.0 | **14.6** | 16.6 | **18.2** | 19.4 | **20.3** |
| 5 mg | 0 | 0.0 | **0.0** | 1.0 | **8.2** | 13.8 | **15.9** | 17.3 | **18.5** |
| **Total** | **0** | **55.6** | **55.8** | **61.6** | **78.4** | **86.0** | **89.6** | **92.3** | **94.4** |

| 25 mg composition | | | | | | | | | |
|---|---|---|---|---|---|---|---|---|---|
| 12.5 mg | 0 | 50.0 | **50.0** | 50.0 | **50.0** | 50.0 | **50.0** | 50.0 | **50.0** |
| 5 mg | 0 | 0.0 | **0.3** | 4.5 | **13.1** | 15.0 | **16.4** | 17.4 | **18.3** |
| 7.5 mg | 0 | 0.0 | **0.0** | 1.3 | **11.1** | 18.6 | **21.4** | 23.4 | **25.0** |
| **Total** | **0** | **50.0** | **50.3** | **55.8** | **74.2** | **83.6** | **87.8** | **90.8** | **93.3** |

| 30 mg composition | | | | | | | | | |
|---|---|---|---|---|---|---|---|---|---|
| 15 mg | 0 | 50.0 | **50.0** | 50.0 | **50.0** | 50.0 | **50.0** | 50.0 | **50.0** |
| 7.5 mg | 0 | 0.0 | **0.3** | 5.7 | **16.4** | 18.7 | **20.5** | 21.8 | **22.9** |
| 7.5 mg | 0 | 0.0 | **0.0** | 1.1 | **9.3** | 15.5 | **17.9** | 19.5 | **20.8** |
| **Total** | **0** | **50.0** | **50.3** | **56.8** | **75.7** | **84.3** | **88.3** | **91.3** | **93.7** |

**Table 12 Bioequivalence analysis of SC injection and Oral MTX**

| BE analysis comparing Otrexup (administered intramuscular or subcutaneous) to oral methotrexate at the 4 doses tested | | | | | |
|---|---|---|---|---|---|
| | Methotrexate Injection **- SC Injection** (Thigh) | | | | |
| Dose Level PK Parameter | Geometric LS Mean | **Oral MTX** Geometric LS Mean | Ratio of Geometric LS Mean(%) | 90% CI for Ratio (%) | Intra-Subject CV (%) |
| Methotrexate 10 mg | | | | | |
| n | 12 | 12 | | | |
| AUC ₍₀₋₂₄₎ (ng•hr/mL) | 1441.5 | 1223.7 | 117.80 | (110.5, 125.6) | 8.9 |
| AUC_{(0-inf)} (ng•hr/mL) | 1470.3 | 1246.9 | 117.91 | (110.7, 125.6) | 8.9 |
| Cₘₐₓ (ng/mL) | 178.4 | 247.2 | 72.17 | (62.6, 83.2) | 20 |

| **Methotrexate 15 mg** | | | | | |
|---|---|---|---|---|---|
| n | 12 | 12 | | | |
| AUC ₍₀₋₂₄₎ (ng•hr/mL) | 1992.7 | 1752.0 | 113.74 | (106.1, 122.0) | 10.0 |
| AUC_{(0-inf)} (ng•hr/mL) | 2040.6 | 1786.6 | 114.22 | (106.3, 122.7) | 10.2 |
| Cₘₐₓ (ng/mL) | 259.9 | 349.4 | 74.38 | (68.2,81.1) | 12.4 |

| **Methotrexate 20 mg** | | | | | |
|---|---|---|---|---|---|
| n | 12 | 12 | | | |
| AUC ₍₀₋₂₄₎ (ng•hr/mL) | 2542.1 | 1927.2 | 131.90 | (120.3, 144.6) | 13.1 |
| AUC_{(0-inf)} (ng•hr/mL) | 2581.8 | 1949.7 | 132.42 | 120.7, 145.3) | 13.2 |
| Cₘₐₓ (ng/mL) | 385.7 | 440.4 | 87.57 | (74.0, 103.6) | 24.5 |

| **Methotrexate 25 mg** | | | | | |
|---|---|---|---|---|---|
| n | 11 | 11 | | | |
| AUC ₍₀₋₂₄₎ (ng•hr/mL) | 2708.6 | 1987.8 | 136.26 | (122.2, 152.0) | 14.4 |
| AUC_{(0-inf)} (ng•hr/mL) | 2745.3 | 2012.4 | 136.42 | (122.4, 152.0) | 14.3 |
| Cₘₐₓ (ng/mL) | 395.9 | 423.5 | 93.47 | (79.06, 110.5) | 22.3 |

## Claims

1. A pharmaceutical composition comprising a plurality of pellets, wherein each pellet comprises methotrexate or a pharmaceutically acceptable salt thereof,
and wherein at least one of the pellets provides immediate release of said methotrexate;
and preferably wherein at least one of the pellets provides delayed release and/or a predetermined lag time prior to release of methotrexate;
and wherein at least 80 % of the total amount of methotrexate of said pharmaceutical composition have been released within 4 hours of administration and/or as measured in an USP Dissolution Apparatus 2 in 500 ml 0.1 N HCI at 37°C ± 0.5°C.

2. A pharmaceutical composition comprising a plurality of pellets, said composition comprising a total dosage of 1 - 30 mg methotrexate or a pharmaceutically acceptable salt thereof, and wherein said composition comprises:
a. At least one pellet A providing immediate release of methotrexate.
b. For the composition comprising more than 10 mg, such as at least 12.5 mg, more preferred 15 mg, preferably 17.5 mg, more preferred 20 mg methotrexate, further at least one pellet B providing delayed release of methotrexate, and preferably
c. For the composition comprising at least 15 mg, preferably 17.5 mg, more preferred 20 mg methotrexate, further at least one pellet C providing delayed release of methotrexate wherein said pellet C provides slower release than said pellet B.

3. A pharmaceutical composition comprising a total amount of methotrexate, or a pharmaceutically acceptable salt thereof, of 15 - 50, preferably 20-30 mg, wherein said pharmaceutical composition releases at least 80% of the total amount of methotrexate within 4 hours; and
wherein said pharmaceutical composition comprises
i) at least one immediate release pharmaceutical composition comprising 10 - 15 mg methotrexate and providing release of at least 8 - 10 mg of methotrexate within 1 hour; and
ii) at least one delayed release pharmaceutical composition comprising a content of methotrexate of at least 2.5 mg, more preferred at least 5 mg methotrexate,
wherein said delayed release pharmaceutical composition releases less than 50% of said content of methotrexate within 1 hour;
preferably each composition comprises one or more pellets, such as one or more tablets.

4. A delayed release pharmaceutical composition comprising a content of methotrexate, or a pharmaceutically acceptable salt thereof, of at least 2.5 mg, more preferred at least 5 mg methotrexate, wherein said delayed release pharmaceutical composition releases less than 50% of said content of methotrexate within 1 hour;
for use in a treatment comprising oral administration of a total amount of methotrexate, or a pharmaceutically acceptable salt thereof, of 15 - 50, preferably 20-30 mg;
wherein said treatment further comprises administration of at least one immediate release pharmaceutical composition comprising 10 - 15 mg methotrexate and providing release of at least 8 - 10 mg of methotrexate within 1 hour,
subject to the proviso than said delayed release pharmaceutical dosage forms and said immediate release pharmaceutical composition releases at least 80% of the total amount of methotrexate within 4 hours;
preferably each composition comprises one or more pellets, such as one or more tablets.

5. An immediate release pharmaceutical composition comprising 10 - 15 mg methotrexate, or a pharmaceutically acceptable salt thereof, and providing release of at least 8 - 10 mg of methotrexate within 1 hour,
for use in a treatment comprising oral administration of a total amount of methotrexate, or a pharmaceutically acceptable salt thereof, of 15 - 50, preferably 20-30 mg;
wherein said treatment further comprises administration of at least one delayed release pharmaceutical composition comprising a content of methotrexate, or a pharmaceutically acceptable salt thereof, of at least 2.5 mg, more preferred at least 5 mg methotrexate, wherein said delayed release pharmaceutical composition releases less than 50% of said content of methotrexate within 1 hour;
subject to the proviso than said delayed release pharmaceutical dosage forms and said immediate release pharmaceutical composition releases at least 80% of the total amount of methotrexate within 4 hours;
preferably each composition comprises one or more pellets, such as one or more tablets.

6. The pharmaceutical composition according to any of the preceding claims, wherein said pellets have a size selected among 0.01 - 5 mm, 0.1 - 4.5 mm, 0.2 - 4 mm, 0.3 - 3.5 mm, 0.4 - 3 mm, 0.5 - 2.5 mm, 0.6 - 2 mm, 0.75 - 1.5 mm, and about 1 mm.

7. The pharmaceutical composition according to any of the preceding claims, wherein at least 50% of the total amount of methotrexate has been released after 1 hour.

8. The pharmaceutical composition according to any of the preceding claims, wherein 60-100% of the total amount of methotrexate has been released after 2 hours.

9. The pharmaceutical composition according to any of the preceding claims, wherein 80-100% of the total amount of methotrexate has been released after 3 hours.

10. The pharmaceutical composition according to any of the preceding claims, wherein 80-100% of the total amount of methotrexate has been released after 4 hours.

11. The pharmaceutical composition according to any of claims 7 - 10, comprising an amount of methotrexate selected among at least 10, 12.5, 15, 20, 25 and 30 mg.

12. The pharmaceutical composition according to any of the preceding claims, wherein said disease is an inflammatory disease or a cancer.

13. The pharmaceutical composition according to any of the preceding claims, wherein said inflammatory disease is selected among Rheumatoid arthritis, juvenile idiopathic arthritis, psoriasis and or psoriasis arthritis and said cancer is selected among Acute Lymphocytic Leukemia or Chronic Lymphocytic Leukemia.

14. The pharmaceutical composition according to any of the preceding claims, wherein said pellets are in a capsule or a sachet.

15. The pharmaceutical composition according to any of the preceding claims, for use in a treatment wherein said composition is administered once a week or twice within 12, preferably 8 hours once a week.
